# FASCICULE DE BREVET EUROPEEN

(11) **EP 3 555 265 B1**
(45) Date de publication et mention de la délivrance du brevet: **25.11.2020**
(21) Numéro de dépôt: 17835636.6
(22) Date de dépôt: 15.12.2017
(51) Int. Cl.: C12N 5/0775, C12N 5/00

(54) **PROCEDE DE CULTURE, D'ISOLEMENT ET D'ENRICHISSEMENT DE CELLULES SOUCHES MESENCHYMATEUSES CLONOGENIQUES, A FORT RENDEMENT, EN VUE D'UNE UTILISATION THERAPEUTIQUE**
VERFAHREN ZUR KULTIVIERUNG, ISOLIERUNG UND ANREICHERUNG VON MESENCHYMALEN KLON-STAMMZELLEN MIT GROSSEM LEISTUNGSPOTENZIAL IM HINBLICK AUF EINEN THERAPEUTISCHEN EINSATZ
HIGH-YIELD METHOD FOR CULTIVATION, ISOLATION AND ENRICHMENT OF CLONOGENIC MESENCHYMATORY STEM CELLS FOR THERAPEUTIC USE

(30) Priorité: 16.12.2016 EP 16306711
(43) Date de publication de la demande: 23.10.2019
(73) Titulaire: Stem Cell Vet Therapeutics, 75018 Paris (FR)
(72) Inventeur: KADRI, Tewfik, 95110 Sannois (FR)
(74) Mandataire: Lavoix
(86) Numéro de dépôt international: PCT/EP2017/083153
(87) Numéro de publication internationale: WO 2018/114718

(56) Documents cités:
- US-A1- 2010 015 705
- US-A1- 2014 314 872
- JAN H. SPAAS ET AL: "Culture and characterisation of equine peripheral blood mesenchymal stromal cells", THE VETERINARY JOURNAL, vol. 195, no. 1, 1 janvier 2013 (2013-01-01), pages 107-113, XP055049712, ISSN: 1090-0233, DOI: 10.1016/j.tvjl.2012.05.006
- GOTTIPAMULA SANJAY ET AL: "Large-scale expansion of pre-isolated bone marrow mesenchymal stromal cells in serum-free conditions", JOURNAL OF TISSUE ENGINEERING AND REGENERATIVE MEDICINE, vol. 10, no. 2, février 2016 (2016-02), pages 108-119, XP002767971,
- NATHALIE MEULEMAN ET AL: "Human marrow mesenchymal stem cell culture: serum-free medium allows better expansion than classical alpha-MEM medium", EUROPEAN JOURNAL OF HAEMATOLOGY, vol. 76, no. 4, 1 avril 2006 (2006-04-01), pages 309-316, XP055107919, ISSN: 0902-4441, DOI: 10.1111/j.1600-0609.2005.00611.x
- Q.Z. ZHANG ET AL: "Human Oral Mucosa and Gingiva", JOURNAL OF DENTAL RESEARCH, vol. 91, no. 11, 1 novembre 2012 (2012-11-01), pages 1011-1018, XP055352995, US ISSN: 0022-0345, DOI: 10.1177/0022034512461016
- MARIA ESTER BERNARDO ET AL: "expansion of mesenchymal stromal cells", BEST PRACTICE & RESEARCH CLINICAL HAEMATOLOGY, vol. 24, no. 1, 1 janvier 2011 (2011-01-01), pages 73-81, XP028177082, ISSN: 1521-6926, DOI: 10.1016/J.BEHA.2010.11.002 [extrait le 2010-11-19]

## Description

La présente invention concerne un procédé d'enrichissement de cellules stromales mésenchymateuses de mammifères en cellules souches multipotentes clonogéniques ou leurs produits dérivés (facteurs sécrétés, microvésicules...) et leurs utilisations thérapeutiques.

### Arrière-plan technique

Les débuts de la thérapie cellulaire utilisant des Cellules dites Stromales Mésenchymateuses (CSM) remontent à plusieurs décennies. La première transplantation de moelle osseuse (MO) en clinique humaine date de 1957, permettant de favoriser la reconstitution hématopoïétique de patients après irradiation accidentelle (1). En 1961 est ensuite réalisée la 1^{ère} transplantation hétérotopique de MO, qui permettra de mettre en évidence le potentiel ostéogénique des cellules médullaires (1). C'est également au cours des années soixante que l'attribution des propriétés de la MO à ses différents constituants cellulaires va progressivement débuter. Alexander Friedenstein découvre ainsi l'existence de progéniteurs multipotents isolés par adhérence au plastique à partir de MO et de rate (2). Il va ensuite mettre en évidence grâce à un modèle de greffe hétérotopique la capacité de cellules fibroblastiques à recréer un environnement hématopoïétique *in vivo* après une étape de culture (3). La présence de Colony-Forming Unit-Fibroblasts (CFU-F) dans les cultures de ces cellules a été décrite en 1980 (4). Enfin, Arnold Caplan a par la suite "popularisé" le terme de Cellule Souche Mésenchymateuse dans le début des années 1990 et montré que ces cellules étaient capables de générer du cartilage, des tendons et du muscle (5). Dans les années 2000, l'insuffisance de données convaincantes permettant d'affirmer le caractère souche des CSM comme les ont définies Loeffer and Potten en 1997 (6) ont conduit l'International Society of Cellular Therapy (ISCT) à proposer la dénomination de Cellules Stromales Mésenchymateuses permettant ainsi de conserver le même acronyme et de mettre en avant leurs capacités trophiques (7,8).

Trois critères minimaux ont été déterminés pour que des cellules de mammifère puissent porter le nom de CSM:
1- Une adhérence au plastique dans des conditions classiques de culture,
2- L'expression d'un panel de marqueurs antigéniques de surface: positives pour le CD73 (ecto-5' nucleotidase, SH3, SH4), CD90 (Thy-1), CD105 (endogline, SH2) et négatives pour un ensemble de marqueurs hématopoïétiques (CD34, CD45, CD11b, CD14, CD19, CD79a, et HLA-DR),
3- La capacité de différenciation *in vitro* vers les voies ostéogénique, chondrogénique et adipocytaire (7).

Des cellules correspondant à ces critères ont depuis été isolées à partir de différents tissus de soutien mésodermiques comme l'os trabéculaire, le liquide synovial, le cartilage, le tissu adipeux, le muscle ou les amygdales, mais aussi à partir de tissus endodermiques comme le thymus ou ectodermiques comme la peau, les follicules pileux et la muqueuse orale (9). Plus récemment les tissus périnataux (gelée de Wharton, membranes placentaires) ont suscité un grand intérêt de par leur accessibilité et l'absence de limites éthiques liées à leur mode de recueil (10,11).

Les capacités de différenciation *in vitro* des CSM ont été les premières à attirer l'attention des cliniciens, conduisant initialement à proposer leur utilisation pour des défauts de réparation de l'appareil musculo-squelettique (5).

Les pathologies les plus explorées en thérapie cellulaire à la fin des années 1990 ont ainsi été les réparations de fractures ou l'ostéogénèse imparfaite caractérisée par un défaut génétique de production de collagène de type I conduisant à une ostéopénie, de multiples fractures, des déformations osseuses graves et une stature considérablement raccourcie (12).

D'autres études dans les années 2000, ont montré que l'injection de CSM autologues suivant une aplasie médullaire consécutive au traitement de patientes atteintes de cancer du sein et ayant déjà reçu une greffe de CSH autologues permettait une sortie plus précoce d'aplasie (13). Il a également été montré par plusieurs équipes que la co-greffe de CSM et de CSH issues du même donneur permettait une meilleure prise de greffe des CSH tout en diminuant le risque d'apparition de réaction du greffon contre l'hôte (GvHD) (14,15). Ces résultats sont à relier au rôle de soutien que jouent les CSM au sein de la niche hématopoïétique. Des études plus récentes ont montré un effet bénéfique de CSM haplo-identiques sur la survie de patients présentant une GvHD sévère de grade IV après allogreffe de CSH (16). Cette équipe postulait pour la première fois que les CSM pouvaient avoir un effet immunosuppresseur *in vivo*. Par la suite d'autres équipes ont commencé à utiliser les CSM pour leurs propriétés sécrétrices cytoprotectrices. Ainsi, l'équipe de Gnecchi en 2005 a montré, dans l'infarctus du myocarde, que l'injection intramyocardique de CSM sur-exprimant le gène Akt avait un effet bénéfique sur la taille de la zone infarcie dès 72 heures après l'injection. Pour confirmer l'hypothèse d'une action paracrine des CSM, des milieux conditionnés issus de cultures de CSM sur-exprimant Akt *in vitro* ont été injectés à des rats soumis à une occlusion coronaire et ont confirmé leur action cardioprotectrice (17,18).

Toutes ces études ont progressivement abouti à l'idée que le mode d'action des CSM était probablement plus lié à la sécrétion de facteurs à action paracrine ayant un effet bénéfique sur les cellules de l'environnement lésionnel plutôt qu'à une différenciation cellulaire permettant de remplacer des cellules lésées. L'efficacité des CSM serait également liée au phénomène dit de "domiciliation lésionnelle" leur permettant après injection de se diriger vers les sites de lésions (19).

Ainsi les différents mécanismes d'action des CSM sont en cours d'exploration, mais certains grands modes de fonctionnement sont déjà bien décrits:
- Le contact direct intercellulaire, la fusion cellulaire, le transfert de mitochondries, l'excrétion de gaz, la synthèse de protéines matricielles,
- Les sécrétions de protéines solubles (cytokines/chimiokines) et le transfert de composants vésiculaires contenant des protéines et microARN (20,21).

Bien que la capacité trophique des CSM ait été décrite dans le maintien de la niche hématopoïétique, il aura fallu quelques années et notamment le travail de l'équipe de Gnecchi pour envisager de mimer l'action thérapeutique des CSM par leurs produits de sécrétions (17). Cet axe thérapeutique a révolutionné le monde de la thérapie cellulaire, mais elle donne également l'occasion d'analyser sous un angle différent les résultats pré-cliniques antérieurs dont l'efficacité avait été exclusivement mise sur le compte de la capacité de différenciation des CSM.

Depuis, de nombreuses études ont mis en évidence le rôle de facteurs solubles produits par les CSM, ces facteurs peuvent être classés selon leur fonctionnalité :

### Facteurs immuno-modulateurs

Dans le début des années 2000, il a été montré que les CSM avaient des capacités immuno-modulatrices *in vitro* dans des réactions lymphocytaires mixtes (22). Par la suite, l'équipe de Di Nicola a démontré, en réalisant des réactions lymphocytaires mixtes avec des CSM soit en contact direct soit en « transwell » (système de chambre avec une membrane semi-perméable laissant passer les facteurs solubles mais séparant physiquement les CSM des autres types cellulaires), que cette capacité immunosuppressive était en partie due à des facteurs solubles (23). L'inhibition de la prolifération lymphocytaire était partiellement restaurée par l'ajout d'anticorps neutralisants anti-Transforming Growth Factor-β1 (TGF-β1) ou anti-Hepatocyte Growth Factor (HGF), suggérant l'implication de ces deux cytokines dans l'action médiée par les CSM (23). L'équipe de Meisel a également montré que lorsque les CSM étaient en présence d'interféron gamma (IFNγ), elles produisaient de l'Indoleamine 2,3-DiOxygenase (IDO), une enzyme catalysant la conversion de tryptophane en kynurénine, et identifiée comme étant un inhibiteur des cellules lymphocytaires T effectrices (24). Plus récemment, certaines équipes se sont intéressées aux fonctions anti-inflammatoires du Tumor Necrosis Factor-Stimulated Gene-6 (TSG-6) qui est activé par le TNFα et l'IL-1 (29). L'équipe de Lee a examiné les facteurs responsables des effets positifs liés à l'injection de CSM dans un modèle d'infarctus du myocarde chez la souris. Ils ont observé que la majorité des cellules se sont logées dans les poumons où le niveau de transcrit de TSG-6 était très augmenté. L'inhibition de TSG-6 entraînait une diminution d'efficacité du traitement par CSM alors que l'apport de TSG-6 exogène permettait de reproduire les effets thérapeutiques liés à l'injection de CSM (30). Depuis cette étude, l'implication de TSG-6 dans l'effet thérapeutique induit par les CSM a été montré dans plusieurs modèles pré-cliniques comme l'ischémie cérébrale, le diabète de type I ou l'encéphalomyélite auto-immune expérimentale (28).

### Facteurs pro-angiogéniques

Les CSM sont capables de produire du Vascular Endothelial Growth Factor (VEGF), mais aussi de l'HGF, du Monocyte Chemotactic Protein 1 (MCP1) et du Stromal cell-Derived Factor 1 (SDF1), facteurs clés du remodelage vasculaire. Une étude de Kinnaird et coll. a montré que des milieux conditionnés de CSM contenant tous les facteurs décrits précédemment ainsi que du basic Fibroblast Growth Factor (bFGF) et du Placental Growth Factor (PIGF), pouvaient favoriser la prolifération des cellules endothéliales et des cellules musculaires lisses. L'addition d'anti-VEGF et anti-bFGF inhibent partiellement ces effets (31). Beaucoup d'études in vivo sur des modèles d'ischémie, indiquent un effet des CSM sur l'augmentation de la densité capillaire ou une amélioration de la perfusion des zones ischémiques, cependant les mécanismes d'action précis des CSM et le rôle potentiel des facteurs trophiques sont encore débattus (20).

### Facteurs anti-apoptotiques

Les MSC synthétisent des protéines inhibitrices de l'apoptose telles que Bcl-2, la survivine, et Akt. Dans un modèle d'insuffisance rénale aiguë, l'équipe de Tögel a montré que les CSM avaient une action protectrice sur la fonction rénale. De plus, ils ont montré que des milieux conditionnés de CSM contenant du VEGF, de l'HGF, et de l'Insulin-like Growth Factor 1 (IGF-1) pouvaient favoriser *in vitro* la croissance et la survie des cellules endothéliales (32). Ces travaux suggèrent que les CSM, en dehors de la production de facteurs directement anti-apoptotiques produisent des molécules comme le VEGF qui participent à l'inhibition de l'apoptose. Cette inhibition passerait par la phosphorylation de Focal Adhesion Kinase (FAK) un puissant signal anti-apoptotique qui passerait par la voie de p53 (20).

### Facteurs chimio-attractifs

Les CSM sont capables de sécréter un grand nombre de chimiokines permettant d'attirer les monocytes, les lymphocytes T, B, NK, les cellules dendritiques mais aussi les progéniteurs hématopoïétiques et endothéliaux (35). Ce recrutement passerait par la sécrétion par les CSM de: CCL2 (MCP-1), CCL3 (MIP-1α), CCL4 (MIP-1β), CCL5 (RANTES), CCL7 (MCP-3), CCL20 (MIP-3α), CCL26 (eotaxin-3), CX3CL1 (fractalkine), CXCL5 (ENA-78), CXCL11 (i-TAC), CXCL1 (GROa), CXCL12 (SDF-1), CXCL8 (IL-8), CXCL2 (GROβ) et CXCL10 (IP-10) (35).

### Facteurs anti-fibrotiques

Meirelles et coll. indiquent dans leur revue de 2009 une action anti-fibrotique des CSM et que dans la plupart des cas, l'injection de CSM était efficace si les cellules étaient administrées avant l'installation d'une fibrose myocardique majeure (39). Cet effet anti-fibrotique serait médiée, chez la souris, par la sécrétion de bFGF et d'HGF dans un modèle de fibrose du tissu adipeux induit par ischémie/reperfusion (40).

### Facteurs antioxydants

Le stress oxydatif se produit en réponse à un stimulus physico-chimique ou physiologique comme l'âge par exemple, provoquant un déséquilibre dans la balance oxydation/anti-oxydation. Il y a alors infiltration des cellules de l'inflammation, relargage de protéases et accumulation d'espèces réactives dérivées de l'oxygène (ROS) à l'origine de nombreuses lésions tissulaires (20). Pour citer un exemple, dans un modèle d'insuffisance pulmonaire aiguë induite par le LPS, l'injection de CSM a permis d'améliorer l'expression de l'enzyme anti-oxydante hème oxygénase-1 (HO-1) et diminuer l'expression de la malondialdehyde (MDA), indicatif d'une peroxydation des lipides (41).

### Facteurs anti-bactérienslanti-parasitaireslanti-viraux

Dans des études récentes menées sur le choc septique d'origine bactérienne il a été montré que de l'administration de CSM permettrait d'améliorer la survie des animaux notamment en favorisant la lyse bactérienne. Lors de cultures de CSM en présence de bactéries Gram-, une analyse des peptides antimicrobiens a permis de montrer que le peptide cathelicidine hCAP-18/LL-37 était responsable de l'activité antimicrobienne médiée par les CSM. *In vivo*, dans un modèle de pneumonie induite par E. Coli, l'administration intra-trachéale de CSM diminue le nombre de colonies formées dans les poumons et le liquide broncho-alvéolaire. Lorsqu'un anticorps neutralisant anti-LL-37 est ajouté, la clairance bactérienne diminue (42).

### Sécrétion de microparticules: exosomes et microvésicules par les CSM

Les Microvésicules (MV) et exosomes sont des microparticules dérivées des cellules qui prennent forme à travers la membrane plasmique. La taille de ces particules varie de 40 à 100nm pour les plus petites d'entre elles (les exosomes) et de 100 à 1000nm pour les plus grosses (les microvésicules). La sécrétion d'exosomes a tout d'abord été appréhendée comme un processus utilisé par la cellule pour se débarrasser de ses déchets. Aujourd'hui, il est admis que les exosomes et MV jouent un rôle très important dans les communications intercellulaires et le transfert de protéines et d'ARN. En 2009, Bruno et coll. ont été les premiers à utiliser *in vitro* des MV issues de CSM dans un modèle d'apoptose cellulaire rénale montrant qu'elle sont capables d'inhiber l'apoptose et stimuler la prolifération des cellules épithéliales tubulaires (45). Zhang et coll. ont démontré que les exosomes dérivés de MSC possèdent des propriétés immunologiques semblables à celles des cellules dont ils sont issus: ils seraient capables d'induire une polarisation macrophagique de type M2 et d'orienter le phénotype d'une population de lymphocytes T CD4+ vers un phénotype de T régulateurs CD4+CD25+FoxP3+ (46). Dans un modèle de greffe d'épiderme chez la souris, l'injection d'exosomes issus de CSM a permis de diminuer le phénomène de rejet grâce à la migration de lymphocytes T régulateurs sur le site lésionnel (46).

Les MV ont également montré une efficacité dans d'autres modèles aussi variés que l'insuffisance pulmonaire aiguë (47), la fibrose hépatique (48), les lésions cérébrales traumatiques (49) ou la cicatrisation cutanée (49). De la même manière que leurs cellules mères, les MV/exosomes dérivés de CSM posséderaient les mêmes propriétés immuno-modulatrices et peuvent être envisagés comme substituts à la thérapie cellulaire pour le traitement de pathologies immunologiques et inflammatoires.

Toutes les études décrites ci-dessus indiquent bien qu'une part importante de l'efficacité des CSM est liée à leur activité paracrine. L'identification de tous ces facteurs a permis une meilleure compréhension de leur fonctionnement et des interactions avec leurs "cibles". Cela a également mis en lumière la sensibilité des CSM à leur environnement dans lequel différents types de stimuli les amènent à produire un panel très varié de facteurs.

En conséquence, un nouvel axe de recherche se développe aujourd'hui visant à optimiser l'utilisation thérapeutique des CSM et/ou de leurs produits de sécrétion en modulant l'environnement de culture, c'est le concept de "priming" cellulaire. Ainsi, l'optimisation des CSM a globalement deux objectifs:
- Les préparer/sensibiliser à l'environnement dans lequel elles vont être injectées *in vivo ;*
- Moduler leur comportement pour contrebalancer ou favoriser une réaction physiologique.

Les modifications de l'environnement peuvent être de différents types: modifications chimiques (molécules pro- ou anti-inflammatoires, facteurs de croissance, agents bactériens), physiques (taux d'oxygène, température, irradiation gamma, UV...), mécaniques (support matriciel) ou par interactions cellulaires (28, 50, 51).

Trois méthodes sont généralement utilisées pour l'isolement des CSM : elles font appel soit aux propriétés antigéniques des cellules, soit à leurs capacités d'adhérence, soit à leur densité cellulaire. La technique la plus courante utilise une sélection par adhérence des CSM au plastique de culture précédée ou non par une sélection de la fraction cellulaire mononucléée par gradient de densité sur Ficoll (Majumdar MK et al. J Cell Physiol 2000 ; 185(1):98-106). Le problème posé par cette technique est l'obtention d'une population hétérogène de cellules fibroblastiques mésenchymateuses avec une faible proportion de CSM. D'autres techniques faisant appel à des sélections immunomagnétiques à partir d'anticorps dirigés contre des marqueurs membranaires (CD271, Stro-1, CD49a par exemple) permettent une purification des CSM mais là encore, n'existant pas de marqueur spécifique de CSM souches, il n'est pas possible d'obtenir une population vraiment enrichie en CSM progénitrices (Gronthos S et al. Blood 1995; Deschaseaux F. et al. Br J Haematol 2003; Quirici N. et al. Exp Hematol 2002).

La demande de brevet US 2014/314872 décrit une méthode de traitement thérapeutique d'animaux, utilisant un surnageant de CSM.

L'article Spaas et al. The Veterinary Journal, 2013, 195(1) : 107-113 décrit la caractérisation de CSM équines isolées de sang périphérique.

L'article Gottipamula et al. Journal of Tissue Engineering and Regenerative medicine, 2016, 10(2), 108-119 décrit le criblage de différents milieu de culture sans sérum pour leur capacité à supporter la croissance et l'expansion de CSM dérivées de moelle osseuse.

L'article Meuleman et al., European Journal of Haematology, 2006, 76(4) : 309-316 compare la capacité de deux milieux à soutenir l'amplification de CSM humaines dérivées de moelle osseuse.

Zhang et al. Journal of Dental Research, 2012, 91(11) : 1011-1018 est une revue sur les CSM humaines dérivées de la muqueuse orale et des gencives.

### Résumé de l'invention

L'invention concerne ainsi un procédé de préparation d'une population cellulaire enrichie en cellules souches mésenchymateuses clonogéniques comprenant :
a) la mise en culture d'une population cellulaire en suspension susceptible de contenir des cellules souches mésenchymateuses clonogéniques, sur un support solide, dans un milieu de culture comprenant du sérum, jusqu'à confluence ;
b) l'élimination du surnageant de culture, le détachement des cellules arrivées à confluence du support solide, et la mise en suspension des cellules dans un milieu de culture dépourvu de sérum ;
c) la mise en culture des cellules obtenues à l'étape b) sur un nouveau support solide et dans un milieu de culture dépourvu de sérum pendant 18h à 30h;
d) le retrait du milieu de culture dépourvu de sérum et l'ajout d'un milieu de culture comprenant sérum ;
e) la culture des cellules dans le milieu de culture comprenant du sérum, pendant au moins 8 jours, de préférence jusqu'à confluence, avec éventuellement renouvellement du milieu de culture supplémenté en sérum ;
f) la récolte des cellules adhérentes obtenues, lesdites cellules adhérentes constituant une population cellulaire enrichie en cellules souches mésenchymateuses clonogéniques par rapport à la population cellulaire mise en culture à l'étape a).

L'invention concerne également un procédé de préparation d'un milieu conditionné de cellules souches mésenchymateuses clonogéniques, comprenant :
a) l'obtention de cellules souches mésenchymateuses clonogéniques par mise en œuvre du procédé selon l'invention,
b) la mise en culture des cellules souches mésenchymateuses clonogéniques sur un support solide et dans un milieu de culture approprié, pendant une durée suffisante pour que les cellules souches mésenchymateuses clonogéniques sécrètent des facteurs ;
c) le remplacement du milieu de culture par du sérum physiologique injectable et le maintien des cellules en culture pendant au maximum 72h ;
d) la récolte du surnageant de culture, celui-ci constituant un milieu conditionné de cellules souches mésenchymateuses clonogéniques.

La divulgation a trait également aux cellules souches mésenchymateuses clonogéniques susceptibles d'être obtenues par un procédé selon l'invention, ainsi que le milieu conditionné de cellules souches mésenchymateuses clonogéniques susceptible d'être obtenu par le procédé selon l'invention.

La divulgation est aussi relative aux cellules souches mésenchymateuses clonogéniques, ou au milieu conditionné de cellules souches mésenchymateuses clonogéniques, pour son utilisation comme médicament.

### Description du brevet

La présente invention se situe dans le domaine des cellules souches adultes. En particulier elle concerne une méthode d'isolement, de sélection, de culture de cellules souches mésenchymateuses clonogéniques ou de leurs produits dérivés (facteurs sécrétés, microvésicules...) en vue d'une utilisation pour le traitement ou la prévention de lésions tissulaires ou de maladies à composante inflammatoire ou dysimmunitaires.

Une des limitations principales pour l'utilisation thérapeutique des CSM est qu'elles sont présentes dans la plupart des tissus en nombre très limité (par exemple dans la moelle osseuse humaine adulte, elles représentent 0,01% des cellules nucléées) si bien que leur isolement, leur purification et leur expansion font appel à des procédés qui peuvent être longs et couteux.

La présente invention découle de la mise en évidence inattendue par les inventeurs qu'un sevrage de sérum pendant 24 heures permet une sélection de cellules souches mésenchymateuses clonogéniques et un meilleur rendement de prolifération des cellules ainsi sélectionnées après remise en culture. Le procédé de l'invention est innovant en ce qu'il inclut une étape d'isolement et de sélection au cours de laquelle une suspension cellulaire issue par exemple de la moelle osseuse, du tissu adipeux, des tissus périnataux ou de la muqueuse orale est cultivée en présence ou non de 10% de sérum de préférence homologue (sérum humain pour cellules d'origine humaine, sérum de cheval pour cellules d'origine équine, sérum de chien pour cellules d'origine canine...); après confluence, les cellules sont passées et remises en culture pendant 24 heures dans un milieu de sélection des cellules les plus immatures, en l'absence de sérum.

La présente invention repose sur une méthode d'isolement et de sélection/enrichissement de cellules souches mésenchymateuses clonogéniques possédant un fort potentiel de prolifération. Cette méthode d'isolement et de culture permet d'obtenir un meilleur rendement de prolifération cellulaire.

Le procédé d'enrichissement selon l'invention comprend la mise en culture d'une population cellulaire en suspension susceptible de contenir des CSM clonogéniques, sur un support solide, dans un milieu de culture comprenant du sérum, jusqu'à confluence. Par exemple les cellules peuvent être ensemencées à une densité d'environ 4000 cellules/cm² et cultivées pendant environ 10 jours pour atteindre la confluence.

Selon l'invention, la population cellulaire en suspension susceptible de contenir des CSM clonogéniques primitives est de préférence une population de cellules primaires issues de biopsie(s) de moelle osseuse, de tissu adipeux, de tissu périnatal, de muqueuse orale, de peau, de muscle, de cartilage, d'amygdale ; d'une ponction de liquide synovial ou de follicules pileux.

Selon un mode de réalisation, le procédé de préparation d'une population cellulaire enrichie en CSM clonogéniques comprend au préalable (avant l'étape a)), la digestion enzymatique d'une biopsie de tissu adipeux, de tissu périnatal, de muqueuse orale, de peau, de muscle, de cartilage, d'amygdale, de manière à obtenir une population cellulaire en suspension susceptible de contenir des cellules souches mésenchymateuses clonogéniques. De préférence, la biopsie est une biopsie de moelle osseuse, de tissu adipeux, de tissu périnatal ou de muqueuse orale. La digestion enzymatique emploie typiquement de la collagénase de manière à libérer les cellules enchâssées dans la matrice extracellulaire. Une biopsie de moelle osseuse peut quant à elle être mise en culture directement, sans digestion enzymatique préalable.

Selon la présente invention, les CSM clonogéniques sont des cellules de mammifère, humaines ou animales, en particulier des cellules humaines, de primates, équines (de cheval en particulier), canines (de chien en particulier), félines (de chat en particulier), ou murines (de souris ou rat en particulier). Il s'agit en outre de cellules d'adultes.

Le procédé de préparation d'une population cellulaire « enrichie » en CSM clonogéniques selon l'invention conduit à l'obtention d'une population cellulaire dans laquelle la concentration et/ou la quantité de CSM clonogéniques est augmentée par rapport à la concentration et/ou à la quantité de CSM clonogéniques dans la population cellulaire de départ, avant mise en œuvre du procédé selon l'invention. Une « cellule souche mésenchymateuse clonogénique » désigne une CSM capable de former une population clonogénique, c'est-à-dire une population de cellules dérivées de la même cellule souche. Une population clonogénique peut comprendre des cellules souches, des cellules progénitrices, des cellules précurseurs et des cellules différenciées, ou n'importe quelle combinaison de celles-ci.

Selon un mode de réalisation, les cellules souches mésenchymateuses clonogéniques sont caractérisées en ce qu'elles présentent les caractéristiques suivantes, aussi bien avant qu'après la mise en œuvre du procédé selon l'invention :
i) une capacité à former des colonies du type CFU-F ;
ii) un phénotype CD44+/CD90+/CD29+ et CD45-/CMH classe II- ; et
iii) une capacité de différenciation ostéoblastique et chondrogénique.

Par « CFU-F » ou « Colony-Forming Unit-Fibroblast », on désigne une cellule clonogénique de stade précoce dans le processus de différenciation des cellules souches mésenchymateuses.

La capacité à former des colonies du type CFU-F est couramment utilisée comme test fonctionnel pour identifier ou dénombrer les CSM dans une population cellulaire hétérogène. Cette capacité est typiquement évaluée en ensemençant une population cellulaire susceptible de contenir des CSM sur un support solide plastique (par exemple plaque de microtitration, flacon, boîte de culture, etc...), dans un milieu de culture approprié (par exemple comprenant un milieu de base MEM Alpha ou DMEM supplémenté par 10% de sérum), et en cultivant les cellules pendant 8 à 14 jours, de préférence 8 à 12 jours. Le nombre de cellules ensemencées est adapté en fonction de la surface de culture et de la durée de culture. A la fin de la culture, les cellules non adhérentes sont éliminées, et les cellules adhérentes sont lavées, par exemple avec du PBS, puis fixées et colorées au crystal violet, avant d'être rincées. Les colonies colorées sont des CFU-F. Seules les colonies de plus de 50 cellules sont prises en compte pour l'identification et/ou le comptage des colonies.

Le phénotype CD44+/CD90+/CD29+ et CD45-/CMH classe II- peut être analysé de manière routinière par l'homme du métier, par exemple à l'aide d'anticorps marqués et analyse par cytométrie en flux.

Les cellules souches mésenchymateuses clonogéniques sont des cellules multipotentes qui peuvent se différencier en un nombre limité de types cellulaires.

Les capacités de différenciation ostéoblastique et chondrogénique sont évaluées par mise en culture des cellules en présence d'inducteurs appropriés et analyse de la différenciation par méthode histochimiques (colorations spécifiques) et/ou par analyse de l'expression de facteurs de transcription clés des différentes voies.

Ainsi pour l'analyse de la capacité de différenciation ostéoblastique, les cellules sont typiquement cultivées en présence d'acide ascorbique et de déxaméthasone pendant 21 jours. La présence de précurseurs ostéoblastiques est révélée par la coloration de Von Kossa montrant les dépôts de Ca2⁺, par la coloration de détection de l'activité phosphatase alcaline, enzyme permettant la minéralisation osseuse, et par le rouge alizarine qui colore la matrice osseuse formée.

Pour l'analyse de la capacité de différenciation chondrogénique, les cellules sont cultivées en présence de TGFβ-3 pendant 21 jours. La présence de précurseurs chondrogéniques est révélée par coloration au bleu alcyan permettant de visualiser au microscope optique les glycosaminoglycanes de la matrice extra-cellulaire produits par les chondrocytes.

Les CSM sont des cellules adhérentes qui ont besoin d'un support solide pour se multiplier et se développer normalement. Elles forment habituellement une couche unicellulaire sur leur support solide dû au phénomène d'inhibition de contact.

Les supports solides utilisés pour la mise en œuvre des procédés selon l'invention sont des supports plastiques (polystyrène). Les supports solides peuvent se présenter sous forme de boîte ou flasque de culture, de plaque de microtitration, ou de microporteurs, ces exemples étant non limitatifs.

Le procédé de préparation d'une population cellulaire enrichie en CSM clonogéniques met en œuvre deux passages cellulaires. Un « passage cellulaire » débute au moment où une suspension de cellules adhérentes est mise en contact avec un support solide dans un milieu de culture et se termine au moment où les cellules adhérentes sont libérées de leur support solide, généralement par traitement enzymatique, et se trouvent à nouveau sous la forme d'une suspension dans le milieu de culture.

Un passage cellulaire comprend habituellement les phases suivantes :
- une phase de colonisation du support solide qui correspond à la période de temps pendant laquelle les cellules qui ont été mises en contact avec le support solide dans un milieu de culture adhèrent au support solide;
- une phase d'amplification des cellules adhérentes au support solide qui correspond à la période de temps pendant laquelle les cellules se multiplient sur le support solide jusqu'à ce que la surface disponible du support solide colonisé soit recouverte à plus de 70%, et de façon préférée à plus de 80%, à plus de 90%, à plus de 95%, ou encore à 100%, par les cellules;
- une phase de détachement des cellules du support solide au moyen d'un traitement enzymatique de sorte qu'un maximum de cellules soient décrochées de leur support (en général plus de 80% et de préférence plus de 90%) dans un espace de temps court (en général en moins de 30 minutes et souvent dans une période de temps inférieure à 20 minutes). La population de cellules est alors essentiellement sous la forme d'une suspension de cellules détachées de leur support solide.

Pendant la phase d'amplification, quand les cellules ont recouvert plus de 90%, de préférence plus de 95%, de préférence encore environ 100%, de la surface disponible du support solide colonisé, on considère que les cellules adhérentes sont « confluentes » ou ont atteint le stade de « confluence ».

Pour le détachement des cellules, le traitement enzymatique emploie généralement une solution contenant une enzyme protéolytique, telle que la trypsine.

Les passages cellulaires successifs sont réalisés en ensemençant un nouveau support solide, dans ou formant un récipient de culture, en utilisant tout ou partie de la population cellulaire obtenue lors du passage cellulaire précédent. On utilise habituellement au moins 80 % de la population cellulaire obtenue lors du passage cellulaire précédent pour réaliser le passage cellulaire suivant. De façon préférée, pour produire une quantité maximale de cellules, on effectue les passages cellulaires successifs en utilisant à chaque fois toute la population cellulaire obtenue lors du passage cellulaire précédent pour réaliser le passage cellulaire suivant.

Le milieu de culture employé dans le cadre de l'invention est un milieu de culture cellulaire conventionnel (« milieu de culture de base »), supplémenté ou non en sérum. Selon l'invention, le milieu de culture de base ne contient ni sérum, ni protéine sérique.

Le milieu de culture de base préférentiellement utilisé pour le procédé de l'invention est le Minimum Essential Medium Alpha (MEM-α). Il est par exemple commercialisé par Biological Industries dépourvu de sérum, de ribonucléosides et de désoxyribonucléosides. D'autres exemples de milieux de culture de base adaptés pour la mise en œuvre du procédé selon l'invention incluent le StemMACS MSC (Miltenyi), DMEM, RPMI.

Pour la mise en œuvre du procédé d'enrichissement selon l'invention, le milieu de culture comprenant du sérum de l'étape a) est un milieu de base de culture, de préférence le MEM-α, supplémenté en sérum, et éventuellement en un ou plusieurs antibiotiques. Selon un mode de réalisation, le milieu de base de culture n'est supplémenté par aucun autre élément que le sérum et éventuellement le ou les antibiotiques. L'ajout d'éléments tel que des cytokines pro-inflammatoires, du lysat plaquettaire ou autre pourraient perturber la prolifération cellulaire.

Selon un mode de réalisation, le sérum est hétérologue aux cellules souches mésenchymateuses, c'est-à-dire qu'il provient d'une espèce différente de celle des CSM (par exemple du sérum de veau fœtal utilisé avec des CSM humaines ou équines). Avantageusement, le sérum est homologue aux cellules souches mésenchymateuses clonogéniques. Autrement dit si les CSM sont humaines, le sérum est du sérum humain ; si les CSM sont des cellules de cheval, le sérum est du sérum de cheval, etc.... Les inventeurs ont en fait découvert que le procédé de l'invention atteint des niveaux d'amplification (c'est-à-dire un nombre de cellules générées) plus élevés lorsqu'il est mis en œuvre avec du sérum homologue. En outre, l'utilisation de sérum homologue permet de s'affranchir du risque de développement par le receveur des cellules, d'anticorps dirigés contre les protéines du sérum d'origine xénogénique.

Avantageusement, le milieu de culture comprenant du sérum de l'étape a) contient 8-12% de sérum (v/v), et en particulier 10% (v/v).

L'étape b) met en œuvre une élimination du surnageant de culture, suivie préférentiellement de un ou plusieurs lavages des cellules adhérentes, le détachement des cellules arrivées à confluence du support solide, puis la mise en suspension des cellules dans un milieu de culture dépourvu de sérum.

A l'issue de l'étape a), les cellules non adhérentes sont en effet éliminées après avoir aspiré le milieu de culture, puis généralement deux rinçages au PBS sont effectués afin d'éliminer les résidus de milieu et le maximum de cellules en suspension. Après les rinçages, le détachement des CSM est effectué.

Après le premier passage cellulaire, les cellules obtenues à l'étape b) sont mises en culture, par exemple à une densité de 3500 à 4500 cellules/cm², typiquement 4000 cellules/cm², sur un nouveau support solide et dans un milieu de culture dépourvu de sérum pendant 18-30h, de préférence 22-26h et de préférence encore pendant 24h pour une sélection optimale des cellules clonogéniques.

Le milieu de culture dépourvu de sérum utilisé aux étapes b) et c) est le milieu de culture de base utilisé à l'étape a), de préférence le MEM-α, éventuellement supplémenté en un ou plusieurs antibiotiques. Selon un mode de réalisation, le milieu de base de culture ne contient ou n'est supplémenté par aucun facteur de croissance ou cytokine. Selon un mode de réalisation, le milieu de base de culture n'est supplémenté par aucun autre élément que le ou les éventuel(s) antibiotique(s).

Une fois la sélection des CSM clonogéniques réalisée, par culture de la population cellulaire en milieu sans sérum, le milieu de culture dépourvu de sérum est retiré puis remplacé par du milieu frais comprenant cette fois-ci du sérum.

Le milieu de culture comprenant du sérum des étapes d)-e) est le milieu de base de culture utilisé aux étapes précédentes, de préférence le α-MEM, supplémenté en sérum homologue ou hétérologue, et éventuellement en un ou plusieurs antibiotiques. Selon un mode de réalisation, le milieu de base de culture n'est supplémenté par aucun autre élément que sérum et éventuellement antibiotiques. Avantageusement, le sérum est homologue aux cellules souches mésenchymateuses clonogéniques.

Avantageusement, le milieu de culture comprenant du sérum des étapes d)-e) contient 8-12% de sérum (v/v), et en particulier 10% (v/v).

La culture des cellules est poursuivie dans le milieu de culture comprenant du sérum, pendant au moins 8 jours, ou encore au moins 10 jours, de préférence jusqu'à confluence, avec éventuellement renouvellement du milieu de culture comprenant du sérum une à deux fois par semaine.

Les cellules adhérentes obtenues à l'issue de l'étape de culture e), sont récoltées à l'étape f). L'étape f) met typiquement en œuvre une élimination du surnageant de culture, suivie préférentiellement de un ou plusieurs lavages des cellules adhérentes (par exemple avec du PBS), le détachement des cellules arrivées à confluence du support solide, puis la mise en suspension des cellules.

Le procédé d'enrichissement selon l'invention peut comprendre en outre, après l'étape f), une étape g) d'isolement des CSM clonogéniques présentes au sein de ladite population cellulaire enrichie en cellules souches mésenchymateuses clonogéniques. Les CSM clonogéniques peuvent être isolées par exemple par marquage phénotypique et tri cellulaire à l'aide d'un appareil de cytométrie en flux.

Pour une production à des fins thérapeutiques, à partir d'une flasque de 300 cm² ensemencée à une densité de 4000 cellules/cm², le procédé selon l'invention permet d'obtenir une population cellulaire enrichie en cellules souches clonogéniques comprenant au moins 30 millions de cellules (contre 15 millions par la mise en œuvre du même procédé mais sans sevrage en sérum).

La population cellulaire enrichie en cellules souches mésenchymateuses clonogéniques, ou les CSM clonogéniques isolées à partir de cette population enrichie peuvent être lavées et mises en suspension dans une solution pour :
i) injection en thérapie cellulaire, en mettant les cellules en suspension dans du sérum physiologique injectable 0.9% NaCl ou un milieu cytoprotecteur comprenant des molécules de la matrice extra-cellulaire (acide hyaluronique, Glycosaminoglycanes...),
ii) Conservation à l'azote, en ajoutant
   - Un agent cryoprotecteur, et éventuellement des glycosaminoglycanes (GAG) ou d'autres agents d'intérêt,
   - Du diméthylsulfoxyde (DMSO, de préférence en qualité injectable ou du glycérol à une concentration de 10% (v/v) ; ou
iii) Lyophilisation.

L'invention concerne également les cellules souches mésenchymateuses clonogéniques directement obtenues par le procédé d'enrichissement selon l'invention. Ces cellules peuvent être sous forme de suspension (congelée ou non), de culture adhérente, ou de lyophilisat.

Le sevrage en sérum mis en œuvre dans le procédé d'enrichissement permet une sélection des cellules mésenchymateuses les plus souches, et conduit à l'obtention d'une population cellulaire finale ayant un profil plus « naïf » sur le plan immunologique (faible expression du CMH II) et aussi plus efficace en termes d'activité anti-inflammatoire (comme caractérisé par exemple par le niveau de sécrétion d'IL-1β par des macrophages activés par le LPS en co-culture avec les CSM), par rapport à des cellules mésenchymateuses obtenues dans les mêmes conditions mais sans sevrage en sérum..

Le rôle de facteurs solubles produits par les CSM, notamment des facteurs immuno-modulateurs, pro-angiogéniques, anti-apoptotiques, chimio-attractifs, anti-fibrotiques, antioxydants, anti-bactériens ou anti-parasitaires ou anti-viraux, et le rôle des exosomes et microvésicules sécrétés/relargués par les CSM, tels que mentionnés dans l'arrière-plan technique, sont bien connus de l'homme du métier.

L'invention concerne donc également un procédé de préparation d'un milieu conditionné de cellules souches mésenchymateuses clonogéniques, comprenant :
a) l'obtention de cellules souches mésenchymateuses clonogéniques par mise en œuvre du procédé d'enrichissement selon l'invention,
b) la mise en culture des cellules souches mésenchymateuses clonogéniques sur un support solide et dans un milieu de culture approprié, pendant une durée suffisante pour que les cellules souches mésenchymateuses clonogéniques sécrètent des facteurs de croissance ;
c) le remplacement du milieu de culture par du sérum physiologique injectable (0,9% NaCl) et le maintien des cellules en culture pendant au maximum 72h ;
d) la récolte du surnageant de culture, celui-ci constituant un milieu conditionné de cellules souches mésenchymateuses clonogéniques.

Les facteurs de croissance sécrétés sont en particulier des facteurs paracrines.

Le surnageant de culture peut ensuite être fractionné, par exemple pour séparer les facteurs solubles et les microparticules sécrétées (exosomes et microvésicules).

Suivant la pathologie dont le traitement est envisagé, ce surnageant de culture à base de sérum physiologique peut être complété avec des glycosaminoglycanes, de l'acide hyaluronique ou des facteurs améliorant la pathologie sélectionnée, comme par exemple des facteurs de croissance plaquettaires (Plasma Riche en Plaquettes : PRP).

Ce procédé mis en œuvre sur une population cellulaire enrichie en CSM clonogéniques permet d'obtenir un surnageant de culture lui-même enrichi en facteurs solubles et microparticules produits par les CSM.

Le milieu conditionné de cellules souches mésenchymateuses clonogéniques directement obtenu par ce procédé, et ses produits dérivés (facteurs solubles et microparticules sécrétées) fait également partie de l'invention.

La demande divulgue également sur les cellules souches mésenchymateuses clonogéniques et/ou le milieu conditionné (ou ses éventuels produits dérivés) pour leur utilisation comme médicament.

La demande divulgue aussi trait à un procédé de traitement thérapeutique d'un sujet qui le nécessite, comprenant l'administration des cellules souches mésenchymateuses clonogéniques et/ou du milieu conditionné selon l'invention audit sujet.

Le sujet est un mammifère, humain ou animal, en particulier humain, primate, équin (cheval en particulier), canin (chien en particulier), félin (chat en particulier), ou murin (souris ou rat en particulier).

Le médicament peut être un médicament de thérapie cellulaire autologue, lorsque les CSM clonogéniques ont été prélevées chez le patient à qui elles vont être administrées. Le médicament peut être un médicament de thérapie cellulaire allogénique, lorsque les CSM clonogéniques ont été prélevées chez un patient différent mais de la même espèce que celui à qui elles vont être administrées.

Les cellules souches mésenchymateuses clonogéniques et/ou le milieu conditionné (ou ses éventuels produits dérivés) ont une utilité particulière pour le traitement des pathologies ostéo-articulaires, des pathologies inflammatoires chroniques et maladies dysimmunitaires, ou d'autres indications thérapeutiques telles que l'hémorragie pulmonaire, la cardiomyopathie dilatée, les ulcères ophtalmiques. Les cellules souches mésenchymateuses clonogéniques et/ou le milieu conditionné (ou ses éventuels produits dérivés) peuvent aussi être utilisés en injection post-opératoire comme anti-inflammatoire, pour le traitement des lésions musculaires ou nerveuses.

### Pathologies ostéo-articulaires et tendineuses :

- Les fractures osseuses quand elles sont traitées de manière conventionnelles réparent généralement bien. Dans certaines situations cliniques comme par exemple lorsqu'il existe une importante perte de substance osseuse, le traitement conventionnel ne permet pas d'obtenir une réparation. Dans ces cas, les CSM, après expansion en culture, avec ou sans étape de pré-différenciation in vitro en ostéoblastes, peuvent être administrées localement dans le foyer de fracture, combinées ou non à des biomatériaux. En situation autologue ou allogénique, les cellules pourraient être isolées et enrichies selon l'invention décrite ci-dessus et injectées in situ dans le foyer de fracture, après ou non pré-différenciation in vitro en ostéoblastes et en combinaison ou non avec des biomatériaux de type hydrogels ou poudre d'hydroxy-apatite.
- Les pathologies articulaires d'origine inflammatoire ou post-traumatiques sont généralement traitées par anti-inflammatoires ou corticoïdes. Quand elles deviennent chroniques, la corticothérapie à long terme peut devenir source de complications à type de dégénérescence tissulaire (ostéoporose, amyotrophie, fragilisation tendineuse...) et doit être stoppée. Les CSM ou leurs produits dérivés (milieu conditionné, exosomes), au regard de leurs propriétés trophiques anti-inflammatoires, cytoprotectrices, pro-angiogéniques pourraient représenter une alternative cellulaire au traitement. En situation autologue ou allogénique, les cellules pourraient être isolées et enrichies selon l'invention décrite ci-dessus et injectées *in situ* dans l'articulation, après ou non pré-différenciation *in vitro* en chondrocytes et en combinaison ou non avec des molécules matricielles de type Glycosaminoglycanes ou acide hyaluronique ou du Plasma Riche en Plaquettes (PRP).
- Les pathologies tendineuses post-traumatiques aiguës ou chroniques sont fréquentes chez les sportifs de haut niveau et leur traitement impose souvent un long repos associé à des anti-inflammatoires administrés localement ou par voie générale. Les CSM ou leurs produits dérivés (milieu conditionné, exosomes), au regard de leurs propriétés trophiques anti-inflammatoires, cytoprotectrices, pro-angiogéniques pourraient représenter une alternative cellulaire au traitement. En situation autologue ou allogénique, les cellules pourraient être isolées et enrichies selon l'invention décrite ci-dessus et injectées *in situ* dans l'articulation, après ou non pré-différenciation *in vitro* en ténocytes et en combinaison ou non avec des molécules matricielles de type Glycosaminoglycanes ou acide hyaluronique ou du Plasma Riche en Plaquettes (PRP).

### Pathologies inflammatoires chroniques et maladies dysimmunitaires :

- Les maladies auto-immunes sont souvent la conséquence d'une réponse immunitaire inappropriée et la cause de nombreuses pathologies médicales chroniques comme le diabète de type I, les maladies immunitaires chroniques de l'intestin (Crohn, Rectocolite hémorragique), la sclérodermie systémique, la sclérose latérale amyotrophique, la gingivostomatite du chat, dermatite atopique, asthme, pathologies ophtalmiques d'origine dysimmunitaire... Il a été démontré que les CSM sont douées de propriétés immunosuppressives et peuvent ainsi être bénéfiques quand elles sont administrées à des patients porteurs de ces pathologies. En situation préférentiellement allogénique, les cellules pourraient être isolées et enrichies selon l'invention décrite ci-dessus et injectées préférentiellement par voie générale.
- Les plaies chroniques cutanées sont le résultat d'un processus de cicatrisation perturbé qui se traduit par une absence d'épidermisation. Les CSM ou leurs produits dérivés (milieu conditionné, exosomes), au regard de leurs propriétés trophiques anti-inflammatoires, cytoprotectrices, pro-angiogéniques pourraient représenter une alternative cellulaire au traitement de ce plaies. En situation autologue ou allogénique, les cellules pourraient être isolées et enrichies selon l'invention décrite ci-dessus et injectées ou déposées *in situ* dans la plaie en combinaison ou non avec des molécules matricielles de type Glycosaminoglycanes ou acide hyaluronique ou du Plasma Riche en Plaquettes.

Les CSM clonogéniques selon l'invention peuvent être administrées de manière conventionnelle, par exemple par voie intraveineuse, par voie topique ou par injection intra lésionnelle ou intra articulaire.

L'invention sera illustrée plus en détail par les figures et exemples qui suivent, sans en limiter la portée.

### FIGURES

**Figure 1** : Description des étapes de la méthode permettant d'isoler et d'enrichir les cellules souches mésenchymateuses d'après la présente invention.
**Figure 2** **:** Comparaison de la numération cellulaire en valeur absolue entre cellules stromales sélectionnées par sevrage 24h (sevrées) et non sélectionnées (non sevrées). Illustre les résultats du tableau 1.
**Figure 3** : Comparaison de la numération cellulaire en moyenne entre cellules stromales sélectionnées par sevrage 24h (sevrées) et non sélectionnées (non sevrées). Illustre les résultats du tableau 1.
**Figure 4** : Comparaison du nombre absolu de clones produits après 10 jours de culture entre les cellules stromales sélectionnées par sevrage 24h et non sélectionnées. Illustre les résultats du Tableau 2.
**Figure 5** : Comparaison du nombre moyen de clones produits après 10 jours de culture entre les cellules stromales sélectionnées par sevrage 24h et non sélectionnées. Illustre les résultats du Tableau 2.
**Figures 6-7** : Caractérisation phénotypique. Phénotypage membranaire par cytométrie de flux des cellules stromales de l'échantillon CHV3. Figure 6 : CHV3 Non sevrées ; Figure 7 : CHV3 Sevrées. Ces résultats montrent que les cellules expriment le même profil phénotypique dans les deux conditions d'isolement : CD45-/CD44+/CD90+/CD29+.
**Figure 8** **:** Pourcentage d'expression du marqueur CMH II par les CSM non sevrées et sevrées en sérum.
**Figure 9** **:** Quantification d'IL1β dans le milieu de co-culture des CSM, non sevrées et sevrées en sérum, avec des macrophages activés par du LPS.

### EXEMPLES

### Exemple 1 : Protocole de sélection des progéniteurs clonogéniques de CSM de muqueuse orale équine par sevrage en sérum.

### Matériel et méthodes

Une biopsie tissulaire d'environ 2 cm² est prélevée au niveau de la muqueuse buccale alvéolaire à l'aide d'un punch à biopsie puis est déposée stérilement dans un flacon contenant du milieu MEM Alpha et un cocktail d'antibiotiques (Amphotéricine à 2,5 µg/ml - Penicilline à 100 UI/ml - Gentamicine à 50 µg/ml) puis transportée à température ambiante au laboratoire. La biopsie est ensuite lavée 2 fois pendant 10 minutes dans un bain de PBS additionné d'Amphotéricine à 2,5 µg/ml + Penicilline à 100 UI/ml + Gentamicine à 50 µg/ml.

### Isolement des cellules

La biopsie est ensuite découpée stérilement en petits morceaux (2mm) à l'aide d'un scalpel et d'une pince stériles. Ces petits morceaux sont transférés dans un tube 50 ml contenant un cocktail d'enzymes + antibiotiques à 37°C pendant 2h30 en agitant toutes les 20 minutes : Dispase II Roche 2,4 UI/ml + Collagénase liberase MTF 100WU/ml + Pénicilline 100 UI/ml + Gentamicine 50 µg/ml + Amphotéricine 2,5 µg/ml.

Après 2h30 de digestion, 10 ml de milieu MEM Alpha contenant la même concentration des 3 antibiotiques précédents sont rajoutés afin de stopper la réaction enzymatique. Puis la suspension est tamisée sur un filtre de 100 µm. Le filtrat est centrifugé à 1500 tr/min pendant 10 min à température ambiante et le culot est repris dans 1 ml de milieu de culture : MEM Alpha (Biological Industries) + Sérum de Cheval (Biowest) 10%, Pénicilline 100 UI/ml, Gentamicine 50 µg/ml, Amphotéricine 2,5 µg/ml.

### Comptage des cellules

Après une bonne homogénéisation de la suspension cellulaire, 20 µl de suspension cellulaire sont déposés dans un puits d'une plaque 96 puits + 20 µl de bleu trypan. Après homogénéisation, 10µL de cette nouvelle suspension (trypan + cellules) sont prélevés et déposés dans une cellule de Malassez pour numération cellulaire.

### Primoculture des cellules (CSM muqueuse)

La suspension cellulaire est ensemencée à 4 000 cellules/cm2 dans du milieu de culture MEM Alpha (Biological Industries) + Sérum de Cheval (Biowest) 10% + Pénicilline 100 UI/ml, Gentamicine 50 µg/ml, Amphotéricine 2,5 µg/ml dans une flasque de culture T75 cm2. Au bout de 48h de culture le milieu est changé avec du milieu neuf dont la concentration est plus faible en Amphotéricine 1 µg/ml. Puis le milieu est changé toutes les 48h.

Un test clonogénique de type CFU-F est réalisé afin de quantifier le nombre de cellules clonogéniques dans la suspension cellulaire initiale et ensuite à chaque passage : 1 000 cellules sont ensemencées dans une flasque T25 cm2 contenant MEM Alpha (Biological Industries) + Sérum de Cheval (Biowest) 10% + Pénicilline 100 UI/ml, Gentamicine 50 µg/ml, Amphotéricine 2,5 µg/ml. Après 10 jours +/- 2 jours de culture, les cellules sont rincées au PBS (Pan Biotech), fixées à l'ethanol 70° et colorées au crystal violet (Sigma Aldrich) dilué au 1/5. Les clones ainsi colorés sont comptés au microscope.

### Passage en P1 et sélection des cellules souches par sevrage de sérum

Les cellules de la primoculture arrivées à confluence après environ 10 jours de culture sont mises en présence de trypsine (EDTA Gibco) pendant 5 minutes à 37°C afin de les détacher du support plastique. L'effet de la trypsine est arrêté avec du milieu MEMalpha + Pénicilline 100 UI/ml, Gentamicine 50 µg/ml, Amphotéricine 1 µg/ml (sans sérum de cheval). Les cellules récupérées sont centrifugées à 1500 tr/min pendant 5 min à température ambiante. Le culot est repris dans 1 ml du milieu MEMalpha + Pénicilline 100 UI/ml, Gentamicine 50 µg/ml, Amphotéricine 1 µg/ml. La suspension cellulaire est bien homogénéisée avant comptage à la cellule de Malassez.

Le comptage des cellules se fait comme indiqué précédemment. Une fois comptées, des cellules sont gardées pour la réalisation d'un test clonogénique CFU-F et pour la réalisation du marquage phénotypique par cytométrie en flux.

Les cellules sont réensemencées à 4000 cellules/cm2 dans du milieu MEM Alpha (Biological Industries) + Pénicilline 100 UI/ml + Gentamicine 50 µg/ml + Amphotéricine 2,5 µg/ml sans Sérum de Cheval. Au bout de 24 heures de culture le milieu est changé avec un milieu MEM Alpha (Biological Industries) + Sérum de Cheval (Biowest) 10% + Pénicilline 100 UI/ml + Gentamicine 50 µg/ml + Amphotéricine 2,5 µg/ml. Le milieu est changé toutes les 48 heures.

A la confluence, les cellules sont détachées à la trypsine et numérées pour évaluer le taux de prolifération.

Un test clonogénique de type CFU-F est réalisé pour quantifier le nombre de cellules clonogéniques sélectionnées par le sevrage : 500 cellules sont ensemencées dans une flasque T25 cm2 avec le même milieu que précédemment.

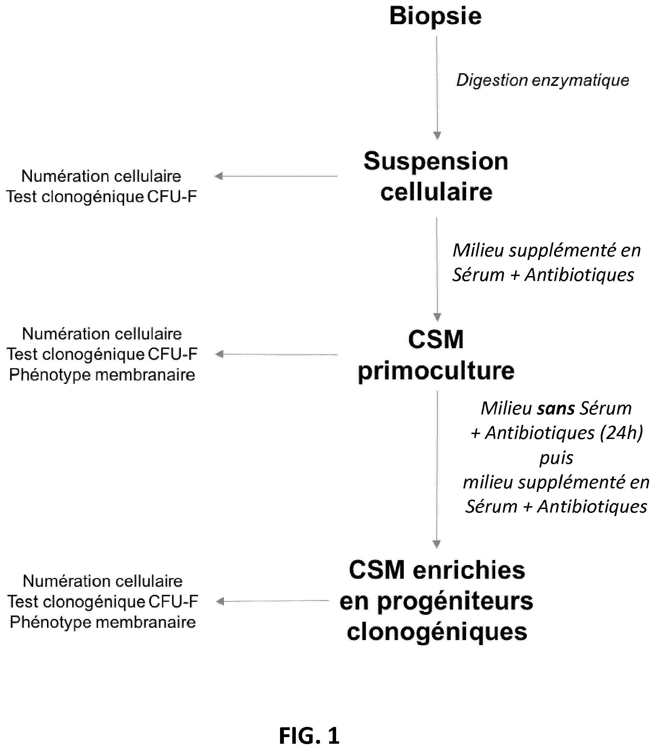

**La** **figure 1** représente les différentes étapes du protocole

### Résultats

A la confluence, en fin de passage P1, les cellules sont détachées à la trypsine et comptées afin d'évaluer le taux de prolifération (**tableau 1**).

**Tableau 1 : Numération cellulaire des cellules stromales non sélectionnées (non sevrées) et sélectionnées par sevrage (sevrées 24h) après 10 jours de culture. Les cellules ont été ensemencées à 4000 cellules/cm². 4 prélèvements issus de muqueuse orale ont été utilisés CHV1, CHV2, CHV3, CHV4.**

| | Non sevré | Sevré 24h | | |
|---|---|---|---|---|
| | Cellules totales comptées | Cellules totales comptées | différence | différence (%) |
| CHV1 | 1425000 | 2614980 | 1189980 | 84 |
| CHV2 | 1570800 | 3480400 | 1909600 | 122 |
| CHV3 | 1412400 | 2085600 | 673200 | 48 |
| CHV4 | 1663200 | 1980000 | 316800 | 19 |
| Moyenne | 1517850 | 2540245 | 1022395 | 67 |

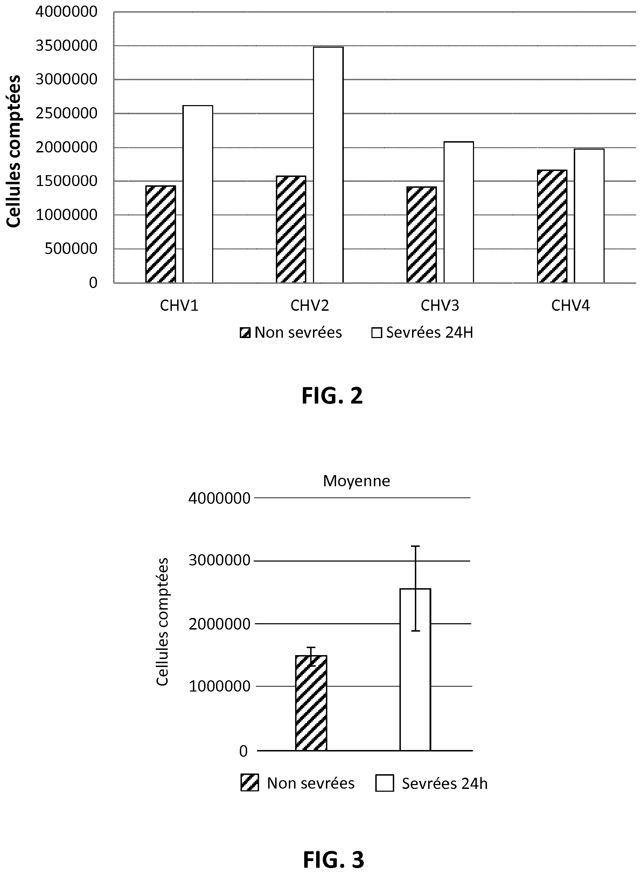

Pour les 4 échantillons, le nombre total de cellules comptées est plus important pour les cellules sevrées en sérum 24h, allant de +19 % à +84 % avec une moyenne de +67%, témoignant d'une prolifération plus importante pour les cellules sélectionnées par sevrage par rapport aux cellules non sevrées (**figures 2 et 3**)

En parallèle de la culture cellulaire, des tests clonogéniques ont été effectués pour déterminer le nombre de CFU-Fs/500 cellules. Les cellules stromales forment des colonies quand elles sont ensemencées à faible concentration après 10 jours de cultures. Le comptage des clones des cellules non sevrées et sevrées se fait à la loupe binoculaire. Ils se caractérisent par la présence de colonies colorées en violet. Au bout de 10 jours de cultures (500 cellules ensemencées par flasque), les colonies formées de plusieurs cellules sont fixées au fond de la flasque et colorées au Crystal violet. Les colonies dont le nombre de cellules est supérieur à 50 sont comptées pour chaque condition au microscope. Un comptage du nombre de colonies permettant d'évaluer les différences de survie des cellules non sevrées et sevrées est effectué **(tableau 2**).

**Tableau 2: Comptage des clones CFU-Fs/500 des cellules stromales non sevrées et sevrées 24 H. Seuls les Clones dont le nombre de cellules est supérieur à 50 sont comptés.**

| | Non sevrées | Sevrées 24h | | |
|---|---|---|---|---|
| | clones > 50 cellules | clones > 50 cellules | différences | différence % |
| CHV1 | 94 | 131 | 37 | 28 |
| CHV2 | 24 | 35 | 11 | 31 |
| CHV3 | 67 | 83 | 16 | 19 |
| CHV4 | 65 | 71 | 6 | 8 |
| Moyenne | 63 | 80 | 18 | 22 |

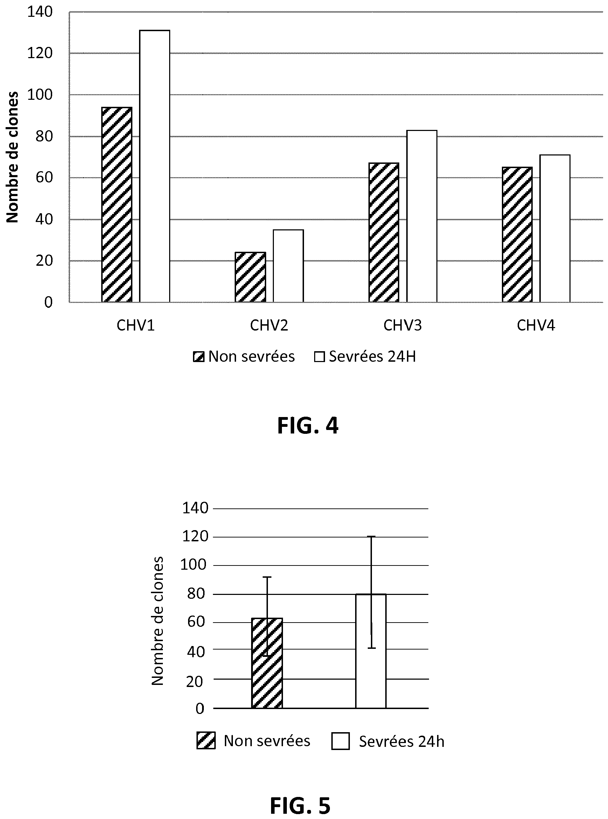

Pour les 4 échantillons, le nombre de colonies est plus important pour les cellules sevrées en sérum 24h, allant de +8% à +28% avec une moyenne de +22%. Cette différence **(****figures 4 et 5**) peut s'expliquer par une plus grande résistance des cellules progénitrices au sevrage en sérum pendant 24h.

Les cellules stromales de la muqueuse orale équine présentent une morphologie similaire aux cellules souches mésenchymateuses trouvées plus communément dans le tissu adipeux ou la moelle osseuse. Les cellules sélectionnées par sevrage lors du passage P1 observées au microscope optique ne montrent pas de différence morphologique en comparaison avec les cellules non sevrées.

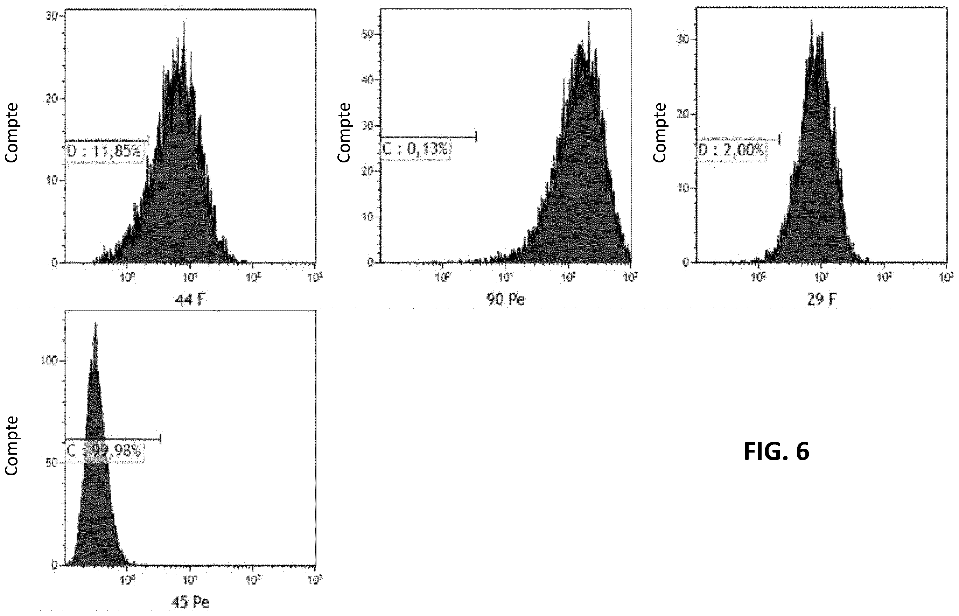

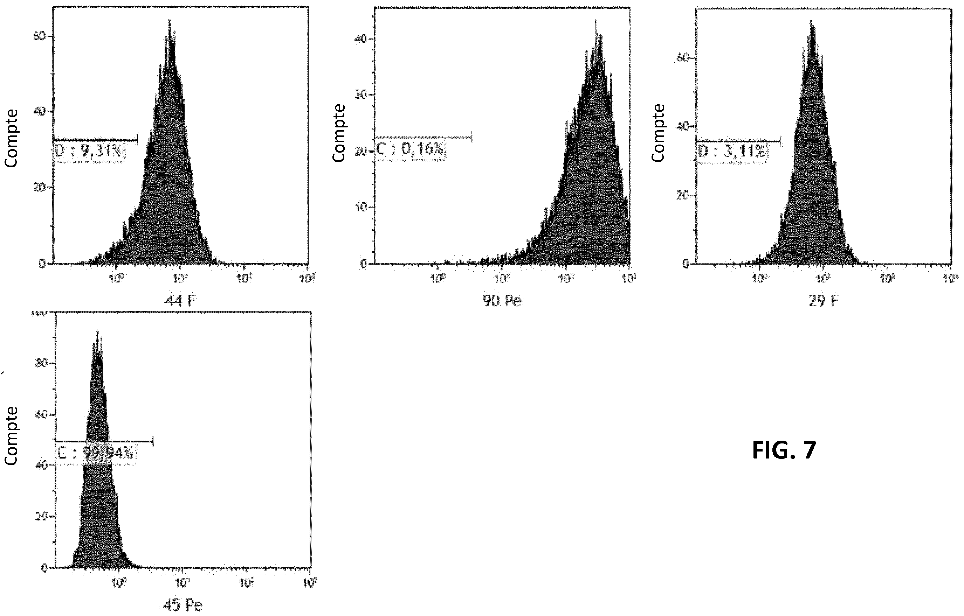

Pour montrer que les cellules cultivées sont bien des cellules stromales mésenchymateuses et que le sevrage n'a pas d'effet sur leur phénotype ou leurs fonctions, différents tests ont été effectués. L'analyse phénotypique par cytométrie en flux **(****figures 6-7****)** montre que les cellules stromales de la muqueuse orale équine ont un phénotype caractéristique des cellules souches mésenchymateuses : CD44+/CD90+/CD29+ et CD45-. Ce phénotype est identique que les cellules aient été sevrées ou non en sérum.

Les cellules stromales mésenchymateuses gardent donc les mêmes caractéristiques phénotypiques après avoir été sélectionnées par sevrage.

Afin de montrer que les cellules stromales utilisées sont bien multipotentes et ont des capacités de différenciation, des tests fonctionnels de différenciation ostéoblastique et de différenciation chondrogénique ont été réalisés sur les cellules stromales de la muqueuse orale équine issues des 4 échantillons. Les méthodes de révélation utilisées (Von Kossa, Rouge alizarine et Phosphatase alcaline pour la capacité fonctionnelle de différenciation ostéoblastique ; coloration au bleu alcyan pour la capacité fonctionnelle de différenciation chondrogénique) montrent que les cellules isolées après sevrage en sérum sont bien capables de se différencier en ostéoblastes et en chondrocytes.

### Exemple 2 : Caractérisation de l'expression des molécules du CMH II par les cellules stromales produites avec ou sans sevrage en sérum

Le Complexe Majeur d'Histocompatibilité de classe II (CMH II) est un système de reconnaissance du « soi » présent à la surface des cellules telles que les monocytes, les macrophages ou bien encore les lymphocytes B. Ces cellules sont appelées « cellules présentatrices d'antigènes » qui ont pour fonction de présenter les molécules antigéniques à une série de lymphocytes T.

Les Cellules Souches Mésenchymateuses (CSM) n'expriment pas le CMH II. Plus cette expression du CMH II est faible, plus les CSM sont « naïves » et ont une immunogénicité faible, permettant ainsi une utilisation clinique beaucoup plus efficace lors d'une thérapie cellulaire allogénique. Certaines études ont montré l'influence de certaines conditions de culture sur l'expression du CMH II par les CSM (52).

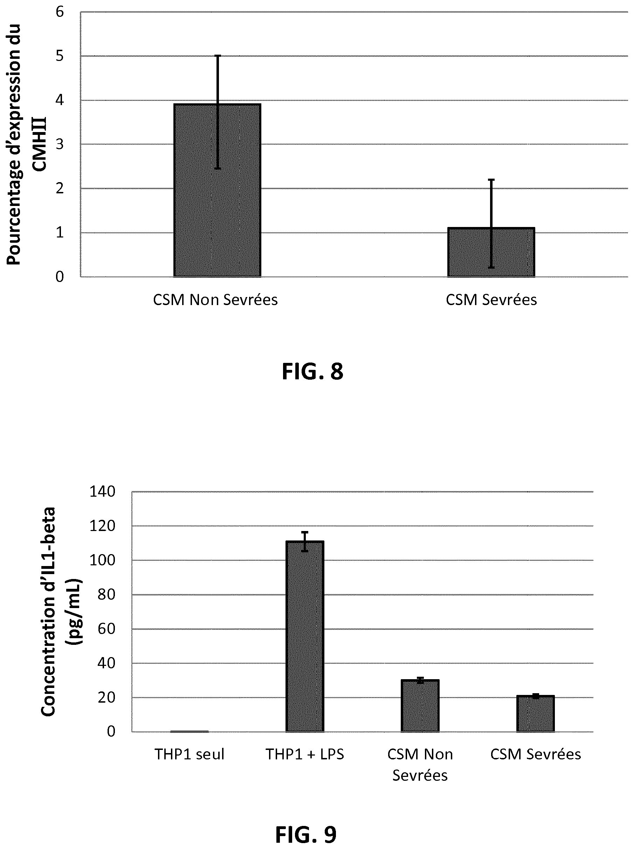

Pour montrer l'influence du sevrage en sérum sur l'expression du CMH II, des CSM équines sevrées et non sevrées en passage P1 (CHV3, CHV4, CHV5) ont été marquées avec un anticorps anti-CMH II spécifique équin. Les CSM ainsi marquées ont ensuite été analysées par cymomètre de flux pour évaluer le niveau d'expression antigénique du CMH II. Les résultats sont représentés sur la **figure 8****.**

Les résultats montrent que les CSM étudiées non sevrées et sevrées expriment faiblement le CMH II (respectivement 3,9 ± 1,3% et 1,1 ± 1,2%). De plus, le pourcentage de cellules sevrées qui expriment le CMH II est significativement plus faible que celui des cellules non sevrées (P= 0,006, n = 6, Test de Student). Les cellules qui expriment le CMH II sont 3 fois moins présentes quand les cellules ont été préalablement sevrées en sérum. Ces résultats suggèrent que le sevrage en sérum, en sélectionnant les cellules les plus « souches », réduit fortement l'immunogénicité des CSM.

### Exemple 3 : Caractérisation de l'activité anti-inflammatoire des cellules stromales produites avec ou sans sevrage en sérum

Des CSM équines de deux chevaux CHV4 et CHV5, non sevrées et sevrées en sérum, ont été mises en culture en présence de macrophages (lignée THP1) activés par du LPS.

L'activation des macrophages par le LPS induit la sécrétion de cytokines pro-inflammatoires telles que l'IL-1β. Les CSM, par leur action immunomodulatrice, sécrètent des facteurs induisant une diminution de synthèse de l'IL-1β par les macrophages.

Les résultats expérimentaux de co-culture des CSM, non sevrées et sevrées en sérum, et des macrophages activés sont donnés dans la **figure 9**. Les résultats montrent que les CSM étudiées, non sevrées et sevrées, ont bien une action immunomodulatrice anti-inflammatoire en agissant sur la synthèse d'IL-1β par les macrophages THP1. La concentration d'IL-1 β baisse fortement en présence de CSM par rapport à la condition témoin « macrophages activés par du LPS seuls ». De plus, l'action des CSM sevrées induit une baisse significative de concentration d'IL 1β plus importante que les cellules non sevrées (20,7 ± 0,4 ng/mL vs. 30 ± 2,4 ng/mL ; P=0,049, n = 6). Ces résultats démontrent une efficacité anti-inflammatoire des cellules sevrées en sérum plus importante que celle des cellules non sevrées en sérum.

En conclusion, le sevrage en sérum, en permettant une sélection des cellules les plus souches, permet d'obtenir un produit cellulaire final à la fois plus « naïf » sur le plan immunologique (faible expression du CMH II) et aussi plus efficace en termes d'activité anti-inflammatoire.

### Références

1. Bianco P. Stem cells and bone: A historical perspective. Bone. 2015 Jan;70:2-9.
2. Friedenstein AJ, Chailakhjan RK, Lalykina KS. The development of fibroblast colonies in monolayer cultures of guinea-pig bone marrow and spleen cells. Cell Prolif. 1970;3(4):393-403.
3. Friedenstein AJ, Chailakhyan RK, Latsinik NV, Panasyuk AF, Keiliss-Borok IV. Stromal cells responsible for transferring the microenvironment of the hemopoietic tissues. Cloning in vitro and retransplantation in vivo. Transplantation. 1974 Apr;17(4):331-40.
4. Castro-Malaspina H, Gay RE, Resnick G, Kapoor N, Meyers P, Chiarieri D, et al. Characterization of human bone marrow fibroblast colony-forming cells (CFU-F) and their progeny. Blood. 1980 Aug;56(2):289-301.
5. Caplan AI. Mesenchymal stem cells. J Orthop Res Off Publ Orthop Res Soc. 1991 Sep;9(5):641-50.
6. Potten CS, Loeffler M. Stem cells: attributes, cycles, spirals, pitfalls and uncertainties. Lessons for and from the crypt. Development. 1990;110(4):1001-20.
7. Dominici M, Le Blanc K, Mueller I, Slaper-Cortenbach I, Marini F., Krause DS, et al. Minimal criteria for defining multipotent mesenchymal stromal cells. The International Society for Cellular Therapy position statement. Cytotherapy. 2006;8(4):315-7.
8. Horwitz EM, Le Blanc K, Dominici M, Mueller I, Slaper-Cortenbach I, Marini FC, et al. Clarification of the nomenclature for MSC: The International Society for Cellular Therapy position statement. Cytotherapy. 2005;7(5):393-5.
9. Kuhn NZ, Tuan RS. Regulation of stemness and stem cell niche of mesenchymal stem cells: Implications in tumorigenesis and metastasis. J Cell Physiol. 2010 Feb;222(2):268-77.
10. Pappa KI, Anagnou NP. Novel sources of fetal stem cells: where do they fit on the developmental continuum? Regen Med. 2009 May;4(3):423-33.
11. Peltzer J, Montespan F, Thepenier C, Boutin L, Uzan G, Rouas-Freiss N, et al. Heterogeneous Functions of Perinatal Mesenchymal Stromal Cells Require a Preselection Before Their Banking for Clinical Use. Stem Cells Dev. 2015 Feb;24(3):329-44.
12. Horwitz EM, Prockop DJ, Fitzpatrick LA, Koo WW, Gordon PL, Neel M, et al. Transplantability and therapeutic effects of bone marrow-derived mesenchymal cells in children with osteogenesis imperfecta. Nat Med. 1999 Mar;5(3):309-13.
13. Koç ON, Gerson SL, Cooper BW, Dyhouse SM, Haynesworth SE, Caplan AI, et al. Rapid hematopoietic recovery after coinfusion of autologous-blood stem cells and culture-expanded marrow mesenchymal stem cells in advanced breast cancer patients receiving high-dose chemotherapy. J Clin Oncol. 2000;18(2):307-307.
14. Lee ST, Jang JH, Cheong J-W, Kim JS, Maemg H-Y, Hahn JS, et al. Treatment of high-risk acute myelogenous leukaemia by myeloablative chemoradiotherapy followed by co-infusion of T cell-depleted haematopoietic stem cells and culture-expanded marrow mesenchymal stem cells from a related donor with one fully mismatched human leucocyte antigen haplotype. Br J Haematol. 2002 Sep;118(4):1128-31.
15. Lazarus HM, Koc ON, Devine SM, Curtin P, Maziarz RT, Holland HK, et al. Cotransplantation of HLA-Identical Sibling Culture-Expanded Mesenchymal Stem Cells and Hematopoietic Stem Cells in Hematologic Malignancy Patients. Biol Blood Marrow Transplant. 2005 May;11(5):389-98.
16. Le Blanc K, Rasmusson I, Sundberg B, Gôtherstrôm C, Hassan M, Uzunel M, et al. Treatment of severe acute graft-versus-host disease with third party haploidentical mesenchymal stem cells. The Lancet. 2004;363(9419):1439-41.
17. Gnecchi M, He H, Liang OD, Melo LG, Morello F, Mu H, et al. Paracrine action accounts for marked protection of ischemic heart by Akt-modified mesenchymal stem cells. Nat Med. 2005;11(4):367-8.
18. Gnecchi M. Evidence supporting paracrine hypothesis for Akt-modified mesenchymal stem cell-mediated cardiac protection and functional improvement. FASEB J. 2006 Apr 1;20(6):661-9.
19. Wise AF, Williams TM, Kiewiet MBG, Payne NL, Siatskas C, Samuel CS, et al. Human mesenchymal stem cells alter macrophage phenotype and promote regeneration via homing to the kidney following ischemia-reperfusion injury. AJP Ren Physiol. 2014 May 15;306(10):F1222-F1235.
20. Liang X, Ding Y, Zhang Y, Tse H-F, Lian Q. Paracrine Mechanisms of Mesenchymal Stem Cell-Based Therapy: Current Status and Perspectives. Cell Transplant. 2014 Sep 15;23(9):1045-59.
21. Prockop DJ, Oh JY. Medical therapies with adult stem/progenitor cells (MSCs): A backward journey from dramatic results in vivo to the cellular and molecular explanations. J Cell Biochem. 2012; 1460-9.
22. Bartholomew A, Sturgeon C, Siatskas M, Ferrer K, Mclntosh K, Patil S, et al. Mesenchymal stem cells suppress lymphocyte proliferation in vitro and prolong skin graft survival in vivo. Exp Hematol. 2002;30(1):42-8.
23. Di Nicola M, Carlo-Stella C, Magni M, Milanesi M, Longoni PD, Matteucci P, et al. Human bone marrow stromal cells suppress T-lymphocyte proliferation induced by cellular or nonspecific mitogenic stimuli. Blood. 2002;99(10):3838-43.
24. Meisel R. Human bone marrow stromal cells inhibit allogeneic T-cell responses by indoleamine 2,3-dioxygenase-mediated tryptophan dégradation. Blood. 2004 Feb 12;103(12):4619-21.
25. Selmani Z, Naji A, Zidi I, Favier B, Gaiffe E, Obert L, et al. Human leukocyte antigen-G5 secretion by human mesenchymal stem cells is required to suppress T lymphocyte and natural killer function and to induce CD4+ CD25highFOXP3+ regulatory T cells. Stem Cells. 2008;26(1):212-22.
26. Selmani Z, Naji A, Gaiffe E, Obert L, Tiberghien P, Rouas-Freiss N, et al. HLA-G is a Crucial Immunosuppressive Molecule Secreted by Adult Human Mesenchymal Stem Cells: Transplantation. 2009 May;87(Supplement):S62-S66.
27. Németh K, Leelahavanichkul A, Yuen PST, Mayer B, Parmelee A, Doi K, et al. Bone marrow stromal cells attenuate sepsis via prostaglandin E2-dependent reprogramming of host macrophages to increase their interleukin-10 production. Nat Med. 2009 Jan;15(1):42-9.
28. Madrigal M, Rao KS, Riordan NH. A review of therapeutic effects of mesenchymal stem cell secretions and induction of secretory modification by différent culture methods. J Transl Med. 2014;12(1):260.
29. Wisniewski HG, Vilcek J. TSG-6: an IL-1/TNF-inducible protein with anti-inflammatory activity. Cytokine Growth Factor Rev. 1997 Jun;8(2):143-56.
30. Lee RH, Pulin AA, Seo MJ, Kota DJ, Ylostalo J, Larson BL, et al. Intravenous hMSCs Improve Myocardial Infarction in Mice because Cells Embolized in Lung Are Activated to Secrete the Anti-inflammatory Protein TSG-6. Cell Stem Cell. 2009 Jul;5(1):54-63.
31. Kinnaird T. Local Delivery of Marrow-Derived Stromal Cells Augments Collateral Perfusion Through Paracrine Mechanisms. Circulation. 2004 Mar 30;109(12):1543-9.
32. Togel F, Weiss K, Yang Y, Hu Z, Zhang P, Westenfelder C. Vasculotropic, paracrine actions of infused mesenchymal stem cells are important to the recovery from acute kidney injury. AJP Ren Physiol. 2007 Jan 2;292(5):F1626-F1635.
33. Li B, Zhang H, Zeng M, He W, Li M, Huang X, et al. Bone marrow mesenchymal stem cells protect alveolar macrophages from lipopolysaccharide-induced apoptosis partially by inhibiting the Wnt/β-catenin pathway: BMSCs protect AMs from LPS-induced apoptosis. Cell Biol Int. 2015 Feb;39(2):192-200.
34. Raffaghello L, Bianchi G, Bertolotto M, Montecucco F, Busca A, Dallegri F, et al. Human Mesenchymal Stem Cells Inhibit Neutrophil Apoptosis: A Model for Neutrophil Préservation in the Bone Marrow Niche. Stem Cells. 2008 Jan;26(1):151-62.
35. Da Silva Meirelles L, Fontes AM, Covas DT, Caplan AI. Mechanisms involved in the therapeutic properties of mesenchymal stem cells. Cytokine Growth Factor Rev. 2009 Oct;20(5-6):419-27.
36. Majumdar MK, Thiede MA, Mosca JD, Moorman M, Gerson SL. Phenotypic and functional comparison of cultures of marrow-derived mesenchymal stem cells (MSCs) and stromal cells. J Cell Physiol. 1998;176(1):57-66.
37. Majumdar MK, Thiede MA, Haynesworth SE, Bruder SP, Gerson SL. Human marrow-derived mesenchymal stem cells (MSCs) express hematopoietic cytokines and support long-term hematopoiesis when differentiated toward stromal and osteogenic lineages. J Hematother Stem Cell Res. 2000 Dec;9(6):841-8.
38. Sugiyama T, Kohara H, Noda M, Nagasawa T. Maintenance of the Hematopoietic Stem Cell Pool by CXCL12-CXCR4 Chemokine Signaling in Bone Marrow Stromal Cell Niches. Immunity. 2006 Dec;25(6):977-88.
39. Da Silva Meirelles L, Fontes AM, Covas DT, Caplan AI. Mechanisms involved in the therapeutic properties of mesenchymal stem cells. Cytokine Growth Factor Rev. 2009 Oct;20(5-6):419-27.
40. Suga H, Eto H, Shigeura T, Inoue K, Aoi N, Kato H, et al. IFATS Collection: Fibroblast Growth Factor-2-Induced Hepatocyte Growth Factor Secretion by Adipose-Derived Stromal Cells Inhibits Postinjury Fibrogenesis Through a c-Jun N-Terminal Kinase-Dependent Mechanism. Stem Cells. 2009 Jan;27(1):238-49.
41. Li J, Li D, Liu X, Tang S, Wei F. Human umbilical cord mesenchymal stem cells reduce systemic inflammation and attenuate LPS-induced acute lung injury in rats. J Inflamm Lond. 2012;9(1):33.
42. Krasnodembskaya A, Song Y, Fang X, Gupta N, Serikov V, Lee J-W, et al. Antibacterial Effect of Human Mesenchymal Stem Cells Is Mediated in Part from Secretion of the Antimicrobial Peptide LL-37. STEM CELLS. 2010 Dec;28(12):2229-38.
43. Gupta N, Krasnodembskaya A, Kapetanaki M, Mouded M, Tan X, Serikov V, et al. Mesenchymal stem cells enhance survival and bacterial clearance in murine Escherichia coli pneumonia. Thorax. 2012 Jun 1;67(6):533-9.
44. Meisel R, Brockers S, Heseler K, Degistirici Ô, Bülle H, Woite C, et al. Human but not murine multipotent mesenchymal stromal cells exhibit broad-spectrum antimicrobial effector function mediated by indoleamine 2, 3-dioxygenase. Leukemia. 2011;25(4):648-54.
45. Bruno S, Grange C, Deregibus MC, Calogero RA, Saviozzi S, Collino F, et al. Mesenchymal Stem Cell-Derived Microvesicles Protect Against Acute Tubular Injury. J Am Soc Nephrol. 2009 May 1;20(5):1053-67.
46. Zhang B, Yin Y, Lai RC, Tan SS, Choo ABH, Lim SK. Mesenchymal Stem Cells Secrete Immunologically Active Exosomes. Stem Cells Dev. 2014 Jun;23(11):1233-44.
47. Zhu Y, Feng X, Abbott J, Fang X, Hao Q, Monsel A, et al. Human Mesenchymal Stem Cell Microvesicles for Treatment of Escherichia coli Endotoxin-Induced Acute Lung Injury in Mice: MSC MV Attenuates ALI in Part Through KGF. STEM CELLS. 2014 Jan;32(1):116-25.
48. Li T, Yan Y, Wang B, Qian H, Zhang X, Shen L, et al. Exosomes Derived from Human Umbilical Cord Mesenchymal Stem Cells Alleviate Liver Fibrosis. Stem Cells Dev. 2013 Mar 15;22(6):845-54.
49. Zhang J, Guan J, Niu X, Hu G, Guo S, Li Q, et al. Exosomes released from human induced pluripotent stem cells-derived MSCs facilitate cutaneous wound healing by promoting collagen synthesis and angiogenesis. J Transl Med [Internet]. 2015 Dec [cited 2015 Mar 20];13(1). Available from: http://www.translational-medicine.com/content/13/1/49
50. Bartosh TJ, Ylöstalo JH, Bazhanov N, Kuhlman J, Prockop DJ. Dynamic compaction of human mesenchymal stem/precursor cells into spheres self-activates caspase-dependent IL1 signaling to enhance secretion of modulators of inflammation and immunity (PGE2, TSG6, and STC1): Activation of IL1-Signaling in MSC Spheres. STEM CELLS. 2013 Nov;31(11):2443-56.
51. Chen H, Min X-H, Wang Q-Y, Leung FW, Shi L, Zhou Y, et al. Pre-activation of mesenchymal stem cells with TNF-α, IL-1β and nitric oxide enhances its paracrine effects on radiation-induced intestinal injury. Sci Rep. 2015 Mar 3;5:8718.
52. Berglund AK, Fisher MB, Cameron KA, Poole EJ, Schnabel LV. Transforming Growth Factor-β2 Downregulates Major Histocompatibility Complex (MHC) I and MHC II Surface Expression on Equine Bone Marrow-Derived Mesenchymal Stem Cells Without Altering Other Phenotypic Cell Surface Markers. Front Vet Sci. 2017;4:84.

## Revendications

1. Procédé de préparation d'une population cellulaire enrichie en cellules souches mésenchymateuses clonogéniques comprenant :
a) la mise en culture d'une population cellulaire en suspension susceptible de contenir des cellules souches mésenchymateuses clonogéniques, sur un support solide, dans un milieu de culture comprenant du sérum, jusqu'à confluence ;
b) l'élimination du surnageant de culture, le détachement des cellules arrivées à confluence du support solide, et la mise en suspension des cellules dans un milieu de culture dépourvu de sérum ;
c) la mise en culture des cellules obtenues à l'étape b) sur un nouveau support solide et dans un milieu de culture dépourvu de sérum pendant 18h à 30h°;
d) le retrait du milieu de culture dépourvu de sérum et l'ajout d'un milieu de culture comprenant sérum ;
e) la culture des cellules dans le milieu de culture comprenant du sérum, pendant au moins 8 jours, de préférence jusqu'à confluence, avec éventuellement renouvellement du milieu de culture supplémenté en sérum ;
f) la récolte des cellules adhérentes obtenues, lesdites cellules adhérentes constituant une population cellulaire enrichie en cellules souches mésenchymateuses clonogéniques par rapport à la population cellulaire mise en culture à l'étape a).

2. Procédé selon la revendication 1, dans lequel ladite population cellulaire en suspension susceptible de contenir des cellules souches mésenchymateuses clonogéniques est une population de cellules primaires issues de biopsie(s) de moelle osseuse, de tissu adipeux, de tissu périnatal, de muqueuse orale, de peau, de muscle, de cartilage, d'amygdale ; d'une ponction de liquide synovial ou de follicules pileux.

3. Procédé selon la revendication 1 ou 2, qui comprend en outre, avant l'étape a), la digestion enzymatique d'une biopsie de tissu adipeux, de tissu périnatal ou de la muqueuse orale de manière à obtenir une population cellulaire en suspension susceptible de contenir des cellules souches mésenchymateuses clonogéniques.

4. Procédé selon l'une quelconque des revendications 1 à 3, dans lequel les cellules souches mésenchymateuses clonogéniques sont **caractérisées en ce qu'**elles présentent :
i) une capacité à former des colonies du type CFU-F ;
ii) un phénotype CD44+/CD90+/CD29+ et CD45-/CMH classe II- ; et
iii) une capacité de différenciation ostéoblastique et chondrogénique.

5. Procédé selon l'une quelconque des revendications 1 à 4, dans lequel le milieu de culture comprenant du sérum de l'étape a) et/ou des étapes d)-e) est un milieu de culture de base MEM-α supplémenté en sérum, et éventuellement en antibiotiques.

6. Procédé selon l'une quelconque des revendications 1 à 5, dans lequel le milieu de culture comprenant du sérum est **caractérisé en ce que** le sérum est homologue aux cellules souches mésenchymateuses clonogéniques.

7. Procédé selon l'une quelconque des revendications 1 à 6, dans lequel le milieu de culture comprenant du sérum contient 8-12% de sérum (v/v).

8. Procédé selon l'une quelconque des revendications 1 à 7, dans lequel le milieu de culture dépourvu de sérum est un milieu de culture de base MEM-α éventuellement supplémenté en un ou plusieurs antibiotiques.

9. Procédé selon l'une quelconque des revendications 1 à 8, dans lequel lesdites cellules souches mésenchymateuses clonogéniques sont des cellules de mammifère, humaines ou animales.

10. Procédé selon l'une quelconque des revendications 1 à 9, dans lequel lesdits supports solides sont des supports plastiques.

11. Procédé selon l'une quelconque des revendications 1 à 10, dans lequel, à l'étape c), les cellules obtenues à l'étape b) sont mises en culture à une densité de 3500 à 4500 cellules/cm².

12. Procédé selon l'une quelconque des revendications 1 à 11, qui comprend, après l'étape f), l'étape g) d'isolement des cellules souches mésenchymateuses clonogéniques présentes au sein de ladite population cellulaire enrichie en cellules souches mésenchymateuses clonogéniques.

13. Procédé selon l'une quelconque des revendications 1 à 12, dans lequel les cellules adhérentes récoltées à l'étape f) ou les cellules souches mésenchymateuses clonogéniques isolées à l'étape g), sont lavées et mises en suspension dans une solution pour injection en thérapie cellulaire, conservation à l'azote ou lyophilisation.

14. Procédé de préparation d'un milieu conditionné de cellules souches mésenchymateuses clonogéniques, comprenant :
a) l'obtention de cellules souches mésenchymateuses clonogéniques par mise en œuvre du procédé selon les revendications 1 à 13,
b) la mise en culture des cellules souches mésenchymateuses clonogéniques sur un support solide et dans un milieu de culture approprié, pendant une durée suffisante pour que les cellules souches mésenchymateuses clonogéniques sécrètent des facteurs ;
c) le remplacement du milieu de culture par du sérum physiologique injectable et le maintien des cellules en culture pendant au maximum 72h ;
d) la récolte du surnageant de culture, celui-ci constituant un milieu conditionné de cellules souches mésenchymateuses clonogéniques.

15. Procédé selon la revendication 14, dans lequel le surnageant de culture récolté est en outre fractionné ou complémenté avec des glycosaminoglycanes, de l'acide hyaluronique ou des facteurs de croissance plaquettaires.

## Patentansprüche

1. Verfahren zur Herstellung einer mit klonogenen mesenchymalen Stammzellen angereicherten Zellpopulation, aufweisend:
a) In-Kultur-Bringen einer suspendierten Zellpopulation, die klonogene mesenchymale Stammzellen enthalten kann, auf einem festen Träger in einem Serum-aufweisenden Kulturmedium, bis zur Konfluenz;
b) Entfernen des Kulturüberstands, Ablösen der Zellen, die Konfluenz vom festen Träger erreicht haben, und In-Suspension-Bringen der Zellen in einem serumfreien Kulturmedium;
c) In Kultur-Bringen der in Schritt b) erhaltenen Zellen auf einem neuen festen Träger und in einem serumfreien Kulturmedium für 18 Stunden bei 30 Stunden°;
d) Entfernen des serumfreien Kulturmediums und Hinzufügen eines Serum-aufweisenden Kulturmediums;
e) Kultivieren der Zellen in dem Serum-aufweisenden Kulturmedium für mindestens 8 Tage, vorzugsweise bis zur Konfluenz, gegebenenfalls mit Erneuerung des Kulturmediums, welches mit Serum ergänzt ist;
f) Ernten der erhaltenen adhärenten Zellen, wobei die adhärenten Zellen eine Zellpopulation bilden, die, verglichen mit der Zellpopulation, die in Schritt a) in Kultur gebracht wurde, mit klonogenen mesenchymalen Stammzellen angereichert ist.

2. Verfahren nach Anspruch 1, wobei die suspendierte Zellpopulation, die klonogene mesenchymale Stammzellen enthalten kann, eine Population von Primärzellen ist, die aus Biopsie(n) von Knochenmark, Fettgewebe, perinatalem Gewebe, Mund-, Haut-, Muskeln-, Knorpel-, Mandelnschleimhaut; aus einer Punktion von Synovialflüssigkeit oder Haarfollikeln erhalten wurden.

3. Verfahren nach Anspruch 1 oder 2, das ferner vor dem Schritt a) den enzymatischen Verdau einer Biopsie von Fettgewebe, perinatalem Gewebe oder Mundschleimhaut aufweist, um eine suspendierte Zellpopulation zu erhalten, die klonogene mesenchymale Stammzellen enthalten kann.

4. Verfahren nach einem der Ansprüche 1 bis 3, wobei die klonogenen mesenchymalen Stammzellen **dadurch gekennzeichnet sind, dass** sie:
i) die Fähigkeit aufweisen, Kolonien vom CFU-F-Typ zu bilden;
ii) einen CD44+ / CD90+ / CD29+ - und CD45- / CMH-Klasse-II-Phänotyp aufweisen; und
iii) eine osteoblastische und chondrogene Differenzierungsfähigkeit aufweisen.

5. Verfahren nach einem der Ansprüche 1 bis 4, wobei das Serum-aufweisende Kulturmedium aus Schritt a) und / oder aus den Schritten d) -e), ein mit Serum, und gegebenenfalls mit Antibiotika, ergänztes MEM-α- basiertes Kulturmedium ist.

6. Verfahren nach einem der Ansprüche 1 bis 5, wobei das Serum-aufweisende Kulturmedium **dadurch gekennzeichnet ist, dass** das Serum zu den klonogenen mesenchymalen Stammzellen homolog ist.

7. Verfahren nach einem der Ansprüche 1 bis 6, wobei das Serum-aufweisende Kulturmedium 8-12% Serum (v/v) enthält.

8. Verfahren nach einem der Ansprüche 1 bis 7, wobei das serumfreie Kulturmedium ein MEM-α-basiertes Kulturmedium ist, das gegebenenfalls mit einem oder mehreren Antibiotika ergänzt ist.

9. Verfahren nach einem der Ansprüche 1 bis 8, wobei die klonogenen mesenchymalen Stammzellen Säugetier-, Menschen- oder Tierzellen sind.

10. Verfahren nach einem der Ansprüche 1 bis 9, wobei die festen Träger Kunststoffträger sind.

11. Verfahren nach einem der Ansprüche 1 bis 10, wobei in dem Schritt c) die aus dem Schritt b) erhaltenen Zellen mit einer Dichte von 3500 bis 4500 Zellen / cm² in Kultur gebracht werden.

12. Verfahren nach einem der Ansprüche 1 bis 11, welches nach dem Schritt f) einen Schritt g) des Isolierens von klonogenen mesenchymalen Stammzellen aufweist, die in der mit klonogenen mesenchymalen Stammzellen angereicherten Zellpopulation vorhanden sind.

13. Verfahren nach einem der Ansprüche 1 bis 12, wobei die in Schritt f) geernteten, anhaftenden Zellen oder die in Schritt g) isolierten, klonogenen mesenchymalen Stammzellen gewaschen und zur Injektion in Zelltherapie, zur Stickstofflagerung oder zur Gefriertrocknung in einer Lösung in Suspension gebracht werden.

14. Verfahren zur Herstellung eines konditionierten Mediums klonogener mesenchymaler Stammzellen, aufweisend:
a) Erhalten klonogener mesenchymaler Stammzellen mittels Ausführens des Verfahrens gemäß den Ansprüchen 1 bis 13,
b) In-Kultur-Bringen der klonogenen mesenchymalen Stammzellen auf einem festen Träger und in einem geeigneten Kulturmedium für eine Zeit, die ausreicht, damit die klonogenen mesenchymalen Stammzellen Faktoren absondern können;
c) Ersetzen des Kulturmediums durch injizierbares physiologisches Serum und Erhalten der Zellen in Kultur für maximal 72 Stunden;
d) Ernten des Kulturüberstands, wobei letzterer ein konditioniertes Medium aus klonogenen mesenchymalen Stammzellen bildet.

15. Verfahren nach Anspruch 14, wobei der geerntete Kulturbestand ferner mit Glykosaminoglykanen, Hyaluronsäure oder Blutplättchenwachstumsfaktoren fraktioniert oder ergänzt wird.

## Claims

1. Method for preparing a cell population enriched with clonogenic mesenchymal stem cells, comprising:
a) culturing a cell population in suspension which may contain clonogenic mesenchymal stem cells on a solid substrate, in a culture medium comprising serum, to confluence;
b) removing the culture supernatant, detaching the cells that have reached confluence from the solid substrate, and suspending the cells in a serum-free culture medium;
c) culturing the cells obtained in step b) on a new solid substrate and in a serum-free culture medium for from 18 hours to 30 hours;
d) removing the serum-free culture medium and adding a culture medium comprising serum;
e) culturing the cells in the culture medium comprising serum for at least 8 days, preferably to confluence, optionally with renewal of the culture medium supplemented with serum;
f) harvesting the adherent cells obtained, said adherent cells constituting a cell population that is enriched with clonogenic mesenchymal stem cells as compared with the cell population cultured in step a).

2. Method according to claim 1, wherein said cell population in suspension which may contain clonogenic mesenchymal stem cells is a population of primary cells obtained from biopsy(biopsies) of bone marrow, fatty tissue, perinatal tissue, oral mucosa, skin, muscle, cartilage or amygdala; from an aspirate of synovial fluid or pilous follicles.

3. Method according to claim 1 or 2, which further comprises, prior to step a), enzymatic digestion of a biopsy of fatty tissue, perinatal tissue or oral mucosa so as to obtain a cell population in suspension which may contain clonogenic mesenchymal stem cells.

4. Method according to any one of claims 1 to 3, wherein the clonogenic mesenchymal stem cells are **characterised in that** they have:
i) a capacity to form colonies of the CFU-F type;
ii) a CD44+/CD90+/CD29+ and CD45-/MHC class II phenotype; and
iii) a capacity for osteoblastic and chondrogenic differentiation.

5. Method according to any one of claims 1 to 4, wherein the culture medium comprising serum of step a) and/or of steps d)-e) is a MEM-α basic culture medium supplemented with serum and optionally with antibiotics.

6. Method according to any one of claims 1 to 5, wherein the culture medium comprising serum is **characterised in that** the serum is homologous with the clonogenic mesenchymal stem cells.

7. Method according to any one of claims 1 to 6, wherein the culture medium comprising serum contains from 8 to 12% serum (v/v).

8. Method according to any one of claims 1 to 7, wherein the serum-free culture medium is a MEM-α basic culture medium optionally supplemented with one or more antibiotics.

9. Method according to any one of claims 1 to 8, wherein said clonogenic mesenchymal stem cells are mammalian, human or animal cells.

10. Method according to any one of claims 1 to 9, wherein said solid substrates are plastics substrates.

11. Method according to any one of claims 1 to 10, wherein, in step c), the cells obtained in step b) are cultured at a density of from 3500 to 4500 cells/cm².

12. Method according to any one of claims 1 to 11, which comprises, after step f), step g) of isolating the clonogenic mesenchymal stem cells present within said cell population enriched with clonogenic mesenchymal stem cells.

13. Method according to any one of claims 1 to 12, wherein the adherent cells harvested in step f) or the clonogenic mesenchymal stem cells isolated in step g) are washed and suspended in a solution for injection in cell therapy, for preservation with nitrogen or for lyophilisation.

14. Method for preparing a conditioned medium from clonogenic mesenchymal stem cells, comprising:
a) obtaining clonogenic mesenchymal stem cells by carrying out the method according to claims 1 to 13,
b) culturing the clonogenic mesenchymal stem cells on a solid substrate and in a suitable culture medium for a period of time sufficient for the clonogenic mesenchymal stem cells to secrete factors,
c) replacing the culture medium with injectable physiological serum and maintaining the cells in culture for not more than 72 hours;
d) harvesting the culture supernatant, the culture supernatant constituting a conditioned medium from clonogenic mesenchymal stem cells.

15. Method according to claim 14, wherein the harvested culture supernatant is furthermore fractionated or supplemented with glycosaminoglycans, hyaluronic acid or platelet-derived growth factors.
